(19) Europäisches Patentamt
European Patent Office
Office européen des brevets

(11) Publication number: **0 264 074 B1**

# (12) EUROPEAN PATENT SPECIFICATION

(45) Date of publication of patent specification: **30.12.92**

(51) Int. Cl.$^5$: **C12N 1/20**, C12P 21/02, C12N 15/70, //(C12N1/20, C12R1:19),(C12P21/02, C12R1:19)

(21) Application number: **87114733.6**

(22) Date of filing: **08.10.87**

## (54) Expression vector for insulin-like growth factor I.

(30) Priority: **09.10.86 JP 240702/86**

(43) Date of publication of application:
**20.04.88 Bulletin 88/16**

(45) Publication of the grant of the patent:
**30.12.92 Bulletin 92/53**

(84) Designated Contracting States:
**AT BE CH DE ES FR GB GR IT LI LU NL SE**

(56) References cited:
**EP-A- 0 155 655**
**EP-A- 0 206 769**
**WO-A-86/05804**

**PROC. NATL. ACAD. SCI. USA, vol. 81, September 1984, pages 5403-5407; B.E. SCHONER et al.: "Role of mRNA translational efficiency in bovine growth hormone expression in Escherichia coli"**

(73) Proprietor: **FUJISAWA PHARMACEUTICAL CO., LTD.**
**3, Doshomachi 4-chome Higashi-ku**
**Osaka-shi Osaka 541(JP)**

(72) Inventor: **Ikuo, Ueda**
**2-11-95, Uenohigashi**
**Toyonaka-shi Osaka-fu(JP)**
Inventor: **Mineo, Niwa**
**7-15, Kirinokuchi Kamiueno-cho**
**Mukou-shi Kyoto-fu(JP)**
Inventor: **Yoshimasa, Saito**
**5-14-10, Higashitokiwadai Toyono-cho**
**Toyono-gun Osaka-fu(JP)**
Inventor: **Yoshinori, Ishii**
**1-33-B-406, Yamadanishi**
**Suita-shi Osaka-fu(JP)**
Inventor: **Tadashi, Kitaguchi**
**1-9-9, Kukuchinishi-cho**
**Amagasaki-shi Hyogo-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

J. BIOCHEM. vol. 101, no. 5, 1987, pages 1281-1288; Y. SAITO et al.: "Direct expression of a synthetic somatomedin C gene in Escherichia coli by use of a two-cistron system"

(74) Representative: **Türk, Gille, Hrabal, Leifert**
**Brucknerstrasse 20**
**W-4000 Düsseldorf 13(DE)**

## Description

This invention relates to a method for the preparation of insulin-like growth factor I (IGF-I). More particularly, it relates to a method for the preparation of methionyl insulin-like growth factor I (Met-IGF-I) using recombinant DNA technology, and vectors for use in the method.

Insulin-like growth factor (IGF) was separated, as an ethanol soluble fraction, from other insulin-like peptides occurring in blood. IGF includes insulin-like growth factor I (IGF-I) and insulin-like growth factor II (IGF-II). It is known that these two factors promote the growth of various tissue cells, and that their biosynthesis and secretion are governed by growth hormones.

IGFs belong to a class of somatomedins which mediate the growth-promoting actions of growth hormones, and may be useful in the treatment of disorders such as dwarfism, osteoporosis, cartilage degeneration, heart and skeletal muscle deterioration, wound and carbohydrate dysfunction. IGF-I is known to be more closely related to growth hormones than IGF-II, and to be the same substance as somatomedin C.

Heretofore, IGF-I has been extracted from human serum in very low yields and with contaminants. Therefore, it would be a significant advance in the art if an efficient means for producing large quantities of pure IGF-I is developed.

Recently, it has become feasible to biosynthesize various biologically active substances in large scale owing to the development of the recombinant DNA technology. Attempts have also been made on the synthesis of IGF-I by such technology.

As is known in the art of recombinant technology, small peptides such as IGF-I (Mr = about 7,000-7,500) are extremely difficult to produce by direct expression because the expressed peptides are decomposed by the action of proteases in host cells. This problem may be overcomed to some extent by expressing the IGF-I in the form of a fused peptide with an appropriate additional peptide. The expressed fused peptide may be subsequently processed in vivo or in vitro to release IGF-I. For instance, Japanese Patent Publication (Kokai) No. 205997/1984 discloses the preparation of IGF-I using recombinant DNA technology. The expression system employed in the publication, which comprises replication origin, promoter, leader sequence, structural gene bearing processing signal, and termination signal, was constructed and transformed into yeast cells. The transformants were then incubated to express pre-IGF-I in the form of a fused peptide, which was then processed and secreted into the medium.

The inventors have also constructed an expression vector capable of expressing IGF-I in the form of a fused peptide in Esherichia coli. The host transformed with the vector was cultured, and the expressed fused peptide was processed by in vitro cleavage using cyanogen bromide or collagenase to obtain IGF-I (Japanese Patent Publication (Kokai) No. 1396/1986).

However, these known methods have a drawback that they require complicated procedures to cleave the fused peptide and separate IGF-I from other components. Therefore, an efficient method for overcoming such drawback has been waited for a long time.

The inventors have found that the cleavage of IGF-I due to the action of proteases occuring in host cells can be obviated when a certain kind of peptide is simultaneously expressed and retained in the host cells together with the desired peptide. This was established using two-cistron expression system in the recombinant DNA technology. This invention is based upon this finding and provides for two-cistronic vectors functional and replicatable in E. coli, which essentially contain DNA sequences encoding IGF-I and protective peptide which is capable of preventing the cellular proteases from decomposing IGF-I.

The invention also provides for the host cell transformed with the two-cistronic expression vector.

The invention further provides for a method for preparing IGF-I by culturing the transformant under appropriate conditions for the expression of IGF-I-encoding gene.

For the purpose of the invention, as disclosed and claimed herein, the following terms are defined below.

IGF-I-gene - structural gene of IGF-I, or DNA sequence encoding IGF-I.

Met-IGF-I - a derivative of IGF-I, which has an additional methionyl residue at N-terminus of IGF-I.

$Amp^R$ - ampicillin-resistant phenotype or gene conferring the same.

$Tet^R$ - tetracycline-resistant phenotype or gene conferring the same.

$Amp^S$ - ampicillin-sensitive phenotype or gene conferring the same.

$Tet^S$ - tetracycline-sensitive phenotype or gene conferring the same.

Cistron - a functional gene unit which at least comprises translation initiating signal (start codon), structural gene encoding a peptide, and translation terminating signal (stop codon).

LH - left half of $\gamma$-interferon

RH - right half of $\gamma$-interferon

Cd - C-domain of IGF-I

α-hANP - α-human atrial natriuretic polypeptide

A polycistronic vector has been disclosed by B.E Schnoer, Proc. Natl. Acad. Sci. USA, 81 5403-5407 (1984). The author constructed a vector having two cistrons, in which the coding region capable of enhancing the expression of bGH (bovin GH) was incorporated into the first cistron, and the coding region for Met-bGH was introduced into the second cistron. The first cistron encodes a polypeptide consisting of 20-25 amino acids, which is useful to prevent the formation of a stem and loop structure of mRNA and to enhance the binding of mRNA of the ribosome.

The two-cistronic expression vector of the present invention contains a DNA sequence encoding a relatively large "protective peptide" (i.e., Mr = about 500-50,000) in the first cistron and IGF-I gene in the second cistron. The protective peptide is specific in its ability to prevent the proteolytic cleavage of IGF-I. As IGF-I is a basic peptide which is usually labile to proteases, the protective peptide is preferably selected from acidic peptides which are capable of forming a complex with IGF-I. Since the molecullar weight of IGF-I is reported to be about 7,000-7,500, it is preferable that the molecular weight of the protective peptide is between about 500-50,000, more preferably about 3,000-15,000, and that the isoelectric point (pI) of the peptide is between 4.0-7.0, more preferably 5.0-6.0. Illustrative protective peptides are peptide Cd (encoded by Fra-B-7 in Fig. 16), peptide LH (encoded by Fra-B-4 in Fig 13) oral Cd-LH fused peptide (encoded by Fra-B-6). In view of pI value, the Cd-LH is the most preferable peptide to use in the present expression vectors.

Illustrative expression vectors of the invention, pCE-SMtrp and pCE-SM3t (Figs. 2.1 and 2.2, respectively), contain a DNA sequence encoding Cd-LH in the first cistron and a DNA sequence encoding IGF-I in the second cistron. These DNA sequences are transcribed, when the vector is incorporated in a suitable host, to polycistronic mRNAs which, in turn, are translated to give a couple of peptides. These two-cistronic vectors of the invention are schematically shown below:

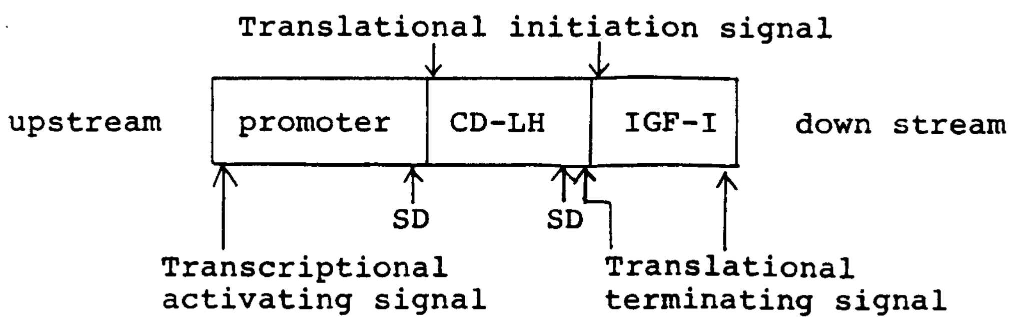


It can be seen from the above illustration that each peptide-encoding gene is preceded by a SD (Shine - Dalgarno) sequence which enhances the translation of the DNA sequence contained in each cistron. The expression vectors of the invention contain the first SD sequence located upstream from the translational initiation signal of the first cistron, and the second SD sequence located upstream from the translational terminating signal of the first cistron. Accordingly, the second SD sequence is a part of the nucleotide sequence encoding the protective peptide.

Therefore, this invention provides for a recombinant DNA expression vectors for the production in E. coli of Met-IGF-I efficiently, said vector comprises in series:

a) a promotor-encoding gene;

b) the first cistron comprising a gene encoding a protective peptide with a molecular weight of about 500-50,000, said gene containing a translational initiation signal immediately adjacent to the codon encoding the N-terminus of said peptide and a translational termination signal positioned downstream from the codon encoding the C-terminus of said peptide;

c) the second cistron comprising a gene encoding IGF-I, said gene containing a translational initiation signal immediately adjacent to the codon encoding the N-terminus of IGF-I and a translational termination signal downstream from the codon encoding the C-terminus of IGF-I; subject to the limitation that said vector contains two SD sequences each located upstream from the codon for the N-terminus of each peptide, and that said vector is selectable and autonomously replicatable in E. coli. The present vector gives Met-IGF-I upon expression, which is as much useful as native IGF-I for medical use.

The nucleotide sequence of the peculiar region where the tranlational termination signal of the first cistron and the translational initiation signal of the second cistron meet together can be more specifically

described as shown below:

```
                                       Translaitonal  Translational
                                       termination    initiation
                                       signal      |  signal
                                                   |    ↓
Lys    Leu    Glu    Glu    Asn    Arg   ↓ Met    Gly    Pro
AAG    CTT    GAG    GAG    AAT  ↑ CGA  TAA TG    GGT  ↑ CCT
 ↑                               |                     |
HindIII           SD            ClaI    Cistron      AvaII
                                        linkage
```

In the above, A is deoxyadenyl, G is deoxyguanyl, C is deoxycytidyl, and T is thymidyl.

As can be seen from the above schema, the "ATG" codon which encodes methionine adjacent to the N-terminus of IGF-I is overlapped with the "TAA" translational termination signal which locates at the 3′ terminus of the nucleotide sequence of the protective peptide. Nucleotide sequences positioned in the region where the translational termination signal of the first cistron and the translational initiation signal of the second cistron meet together is not limited to the above particular sequence, and other sequences are available, such as TGATG, or ATGA in which the order of the termination codon and the initiation codon is reversed, or TAGATG, TAAATG or TGAATG in which the termination codon and initiation codon are located in alignment. As shown in the above schema, the SD sequence for the second cistron may be a part of the nucleotide sequence encoding a protective peptide. In such a case, the SD sequence is preferably positioned 5-15 bp, preferably 6-8 bp upstream from the coding region of translational termination and initiation signals. This can be accomplished using appropriately constructed DNA linkers, such as Fra-A-3, described hereinafter.

This DNA linker, Fra-A-3, was ligated to the 5′ terminus of the IGF-I gene to obtain a DNA fragment (Fra-A-7), which was then ligated into the 4.5 kbp HindIII-BamHI restriction fragment of α-hANP expression vector pCLaHtrpSd (Japanese Patent Publication (Kokai) No. 141900/1986) to form two-cistronic plasmid pCE-SMtrp. Alternatively, the Fra-A-7 was ligated into the 4137 bp HindIII-BamHI restriction fragment of plasmid pCLaHtrp3t (prepared in Preparation 11) to form two-cistronic plasmid pCE-SM3t. When one of these plasmids is transformed into host cells, the resultant transformants produce both peptides simultaneously. This results in safe maintenance of intact Met-IGF-I peptide within the host cells owing to the complex formation between the basic peptide, Met-IGF-I, and the acidic peptide, Cd-LH. Thus, the complex shows resistance to cellular proteases. The isolation of Met-IGF-I from the transformants can be easily performed using conventional methods. For example, after the cultured cells are harvested and lyzed, the lysates are subjected to an appropriate process suitable for the separation of Met-IGF-I from Cd-LH on the basis of the difference in physical properties, such as solubility in acidic solvent.

For convenience of expression of Met-IGF-I in E. coli, plasmid, pBR322 was used as a starting material for the construction of two-cistronic vectors of the invention. However, as those skilled in the art will recognize, many other plasmids, such as pUC9 or pAT153, can be used instead of pBR322 so long as they are capable of replicating in E. coli. Bacteriophages can also be used for the construction of the vectors of the invention.

The construction of the vectors of the invention does not require the use of any specific promoter, and it can be selected from those commonly used. The illustrative vectors of the invention comprise one or more artificial promoters which were synthesized based on the nucleotide sequence of the native promoter of tryptophan operon. For the purpose of the invention, three artificial trp promoters were synthesized. The transcription activating sequences of these three synthetic promoters were same and identical, but the sequence of the 3′ terminal region differed from each other. These were designated "trp promoter I", "trp promoter II" and "trp promoter III".

Transcription termination sequence may be derived from the starting material, i.e., phages or plasmids used for the construction of the present vectors. Alternatively, a synthesized sequence, for example, synthesized fd phage terminator may be used as illustrated in working examples.

For the convenience of the description, the term "synthesized" or "artificial" will be omitted hereinafter in connection with DNA fragments that code for peptides, trp promoters, or fd phage terminator.

Detailed Description of the Invention

The starting plasmid pBR322 containing $Tet^R$ and $Amp^R$ was digested with restriction enzymes EcoRI and BamHI and the resultant large DNA restriction fragment was isolated, which was then ligated to trp promoter II (Fra-B-1, 163 bp) to form plasmid pTrpEB7 which lacks for $Tet^R$. Plasmid pTrpEB7 was digested with restriction enzymes EcoRI and BamHI, and the resultant large fragment containing the trp promoter I, which comprises nucleotide sequence corresponding to the sequence positioned upstream from EcoRI site of Trp promoter II, was obtained. To the DNA fragment was inserted the small EcoRI-BamHI restriction fragment of plasmid pBR322 to form plasmid pBR322trp comprising trp promoter I, $Tet^R$ and $Amp^R$. Trp promoter III (Fra-B-3) was ligated into the EcoRI - ClaI restriction fragment of plasmid pBR322trp to form plasmid pBR322dtrpS.

The nucleotide sequence of trp promoters II and III (Fra-B-1 and Fra-B-3) are as follows.

Fra-B-1 (163 bp) Synthetic promoter II gene

EcoRI*

```
 AATTTGCCGACATCATAACGGTTCTGGCAAATATTCTGAAATGAGC-
     ACGGCTGTAGTATTGCCAAGACCGTTTATAAGACTTTACTCG-
```

HpaI

```
TGTTGACAATTAATCATCGAACTAGTTAACTAGTACGCAAGTTCACGTAAA-
ACAACTGTTAATTAGTAGCTTGATCAATTGATCATGCGTTCAAGTGCATTT-
```

EcoRI

```
AAGGGTATCGAATTCATGGCTGGTTGTAAGAACTTCTTTTGGAAGACTTTC-
TTCCCATAGCTTAAGTACCGACCAACATTCTTGAAGAAAACCTTCTGAAAG-
```

BamHI

```
ACTTCGTGTTGATAG
TGAAGCACAACTATCCTAG
```

Fra-B-3 (105 bp) Synthetic trp promoter III gene

EcoRI*

```
 AATTTGCCGACATCATAACGGTTCTGGCAAATATTCTGAAATGAGCTGTTGA-
     ACGGCTGTAGTATTGCCAAGACCGTTTATAAGACTTTACTCGACAACT-
```

HpaI ClaI

```
CAATTAATCATCGAACTAGTTAACTAGTACGCAAGTTCACGTAAAAAGGGTAT
GTTAATTAGTAGCTTGATCAATTGATCATGCGTTCAAGTGCATTTTTCCCATAGC
```

A schematic illustration of the construction protocol for each of the intermediate plasmid pTrpEB7 and plasmid pBR322dtrpS encoding trp promoter I and III is presented respectively in Fig. 3 and 4 of the accompanying drawings.

Plasmid pCdγ which codes for a fused peptide of Cd and LH-RH was constructed as follows.

LH-encoding gene (Fra-B-4) was ligated into the EcoRI-BamHI restriction fragment of plasmid pBR322 to form plasmid pLH107. Plasmid pLH107 was digested with restriction enzymes HindIII and BamHI to delete the nucleotide sequence of Fra-B-4 located downstream from the HindIII site. RH gene (Fra-B-5) was then ligated into the resulting DNA fragment to form plasmid pγF-3, from which EcoRI-BamHI restriction fragment, i.e. LH-RH-encoding gene (Fra-B-6), was isolated.

The LH-RH-encoding gene (Fra-B-6) was ligated into the EcoRI-BamHI restriction fragment of plasmid

pTrpEB7 to form plasmid pγtrp which contains the LH-RH-encoding gene located downstream from the trp promoter I. The large HpaI-EcoRI restriction fragment of plasmid pγtrp was ligated to a DNA fragment containing part of trp promoter III and Cd-encoding gene (Fra-B-7) to form plasmid pCd γ which comprises trp promoter I and trp promoter III and Cd- and LH-RH- encoding genes in this order. The nucleotide and amino acids sequences of Fra-B-4, Fra-B-5, Fra-B-6, and Fra-B-7 are shown bellow. The illustrative construction protocol of plasmid pCdγ is presented in Fig. 5.

Fra-B-4 (236 bp) LH gene

```
                 EcoRI     1
                                Met Cys Tyr Cys Gln Asp Pro Tyr
                         AATTC-ATG-TGT-TAC-TGC-CAG-GAC-CCA-TAT-
                             G-TAC-ACA-ATG-ACG-GTC-CTG-GGT-ATA-

        10                                      20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                  30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                           HindIII   60
Ser Gln Ile Val Ser Phe Tyr Phe Lys Leu Phe Lys Asn Phe
AGC-CAA-ATT-GTC-TCC-TTT-TAC-TTC-AAG-CTT-TTC-AAA-AAC-TTT-
TCG-GTT-TAA-CAG-AGG-AAA-ATG-AAG-TTC-GAA-AAG-TTT-TTG-AAA-

                            70         stop stop stop BamHI
Lys Asp Asp Gln Ser Ile Gln Lys Ser Val
AAG-GAT-GAC-CAG-AGC-ATC-CAA-AAG-AGT-GTG-TAA-TGA-TAG
TTC-CTA-CTG-GTC-TCG-TAG-GTT-TTC-TCA-CAC-ATT-ACT-ATCCTAG
```

Fra-B-5 (270 bp) RH gene

```
                                       HindIII   60
                                             Phe Lys
                                          AG-CTT-TTC-AAA-
                                              A-AAG-TTT-

                                  70
Asn Phe Lys Asp Asp Gln Ser Ile Gln Lys Ser Val Glu Thr
AAC-TTT-AAG-GAT-GAC-CAG-AGC-ATC-CAA-AAG-AGT-GTG-GAG-ACC-
TTG-AAA-TTC-CTA-CTG-GTC-TCG-TAG-GTT-TTC-TCA-CAC-CTC-TGG-

                80
Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

 90                                     100
Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

                           110
Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

       120                                        130
Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

                                  140
Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-
```

```
    stop BamHI
Gln
CAG-TAA-G
GTC-ATT-CCTAG
```

Fra-B-6 (450 bp) LH-RH gene

```
                    EcoRI    1
                             Met Cys Tyr Cys Gln Asp Pro Tyr
                        AATTC-ATG-TGT-TAC-TGC-CAG-GAC-CCA-TAT-
                             G-TAC-ACA-ATG-ACG-GTC-CTG-GGT-ATA-

        10                                          20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                    30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                    40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                               HindIII   60
Ser Gln Ile Val Ser Phe Tyr Phe Lys Leu Phe Lys
AGC-CAA-ATT-GTC-TCC-TTT-TAC-TTC-AAG-CTT-TTC-AAA-
TCG-GTT-TAA-CAG-AGG-AAA-ATG-AAG-TTC-GAA-AAG-TTT-
```

```
                                    70
Asn Phe Lys Asp Asp Gln Ser Ile Gln Lys Ser Val Glu Thr
AAC-TTT-AAG-GAT-GAC-CAG-AGC-ATC-CAA-AAG-AGT-GTG-GAG-ACC-
TTG-AAA-TTC-CTA-CTG-GTC-TCG-TAG-GTT-TTC-TCA-CAC-CTC-TGG-

                        80
Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

 90                                        100
Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

                              110
Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

            120                                     130
Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

                                    140
Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-

    stop BamHI
Gln
CAG-TAA-G
GTC-ATT-CCTAG
```

Fra-B-7 (124 bp) Peptide Cd gene with a part of DNA fragment of synthetic trp promoter III gene

```
HpaI                                      ClaI      Met Phe Tyr Phe
  AACTAGTACGCAAGTTCACGTAAAAAGGGTATCGATAAA-ATG-TTC-TAC-TTC-
  TTGATCATGCGTTCAAGTGCATTTTTCCCATAGCTATTT-TAC-AAG-ATG-AAG-

Asn Lys Pro Thr Gly Tyr Gly Ser Ser Ser Arg Arg Ala Pro Gln
AAC-AAA-CCG-ACC-GGC-TAT-GGC-TCC-AGC-TCT-CGT-CGC-GCA-CCG-CAG-
TTG-TTT-GGC-TGG-CCG-ATA-CCG-AGG-TCG-AGA-GCA-GCG-CGT-GGC-GTC-

                                         EcoRI
Thr Gly Ile Val Asp Glu Gly Gly Asp Glu Phe
ACT-GGT-ATC-GTA-GAC-GAG-GGT-GGC-GAT-G
TGA-CCA-TAG-CAT-CTG-CTC-CCA-CCG-CTA-CTT-AA
```

Plasmid pCLaHtrpSd which encodes a fused protein of Cd-LH and α-hANP was constructed as follows.

The ClaI-BamHI restriction fragment of plasmid pCdγ (Fra-B-8) was inserted into the ClaI-BamHI restriction site of plasmid p322dtrpS to form an expression plasmid pCdγtrpSd for a fused peptide of Cd and LH-RH.

The α-hANP-encoding DNA with linker DNA (Fra-B-9) was ligated into HindIII-BamHI restriction fragment of plasmid pCdγtrpSd to form an expression vector pCLaHtrpSd encoding Cd-LH-α-hANP fused peptide. Fra-B-8 and Fra-B-9 are shown below. The illustrative construction protocol of plasmids pCdγtrpSd and pCLaHtrpSd are presented in Fig. 6.

Fra-B-8 (542 bp) Cd-LH-RH gene

```
  ClaI      Met Phe Tyr Phe Asn Lys Pro Thr
     CGATAAA-ATG-TTC-TAC-TTC-AAC-AAA-CCG-ACC-
        TATTT-TAC-AAG-ATG-AAG-TTG-TTT-GGC-TGG-

Gly Tyr Gly Ser Ser Ser Arg Arg Ala Pro Gln Thr Gly Ile Val
GGC-TAT-GGC-TCC-AGC-TCT-CGT-CGC-GCA-CCG-CAG-ACT-GGT-ATC-GTA-
CCG-ATA-CCG-AGG-TCG-AGA-GCA-GCG-CGT-GGC-GTC-TGA-CCA-TAG-CAT-

                        EcoRI
Asp Glu Gly Gly Asp Glu Phe Met Cys Tyr Cys Gln Asp Pro Tyr
GAC-GAG-GGT-GGC-GAT-GAA-TTC-ATG-TGT-TAC-TGC-CAG-GAC-CCA-TAT-
CTG-CTC-CCA-CCG-CTA-CTT-AAG-TAC-ACA-ATG-ACG-GTC-CTG-GGT-ATA-

        10                                          20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                    30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                    40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

 50                               HindIII   60
Ser Gln Ile Val Ser Phe Tyr Phe Lys Leu Phe Lys
AGC-CAA-ATT-GTC-TCC-TTT-TAC-TTC-AAG-CTT-TTC-AAA-
TCG-GTT-TAA-CAG-AGG-AAA-ATG-AAG-TTC-GAA-AAG-TTT-
```

```
                                    70
Asn Phe Lys Asp Asp Gln Ser Ile Gln Lys Ser Val Glu Thr
AAC-TTT-AAG-GAT-GAC-CAG-AGC-ATC-CAA-AAG-AGT-GTG-GAG-ACC-
TTG-AAA-TTC-CTA-CTG-GTC-TCG-TAG-GTT-TTC-TCA-CAC-CTC-TGG-

                80
Ile Lys Glu Asp Met Asn Val Lys Phe Phe Asn Ser Asn Lys
ATC-AAG-GAA-GAC-ATG-AAT-GTC-AAG-TTT-TTC-AAT-AGC-AAC-AAA-
TAG-TTC-CTT-CTG-TAC-TTA-CAG-TTC-AAA-AAG-TTA-TCG-TTG-TTT-

 90                                         100
Lys Lys Arg Asp Asp Phe Glu Lys Leu Thr Asn Tyr Ser Val
AAG-AAA-CGT-GAT-GAC-TTC-GAA-AAG-CTG-ACT-AAT-TAT-TCG-GTA-
TTC-TTT-GCA-CTA-CTG-AAG-CTT-TTC-GAC-TGA-TTA-ATA-AGC-CAT-

                          110
Thr Asp Leu Asn Val Gln Arg Lys Ala Ile His Glu Leu Ile
ACT-GAC-TTG-AAT-GTC-CAA-CGC-AAA-GCA-ATA-CAT-GAA-CTC-ATC-
TGA-CTG-AAC-TTA-CAG-GTT-GCG-TTT-CGT-TAT-GTA-CTT-GAG-TAG-

       120                                        130
Gln Val Met Ala Glu Leu Ser Pro Ala Ala Lys Thr Gly Lys
CAA-GTG-ATG-GCT-GAA-CTG-TCG-CCA-GCA-GCT-AAA-ACA-GGG-AAG-
GTT-CAC-TAC-CGA-CTT-GAC-AGC-GGT-CGT-CGA-TTT-TGT-CCC-TTC-

                                    140
Arg Lys Arg Ser Gln Met Leu Phe Gln Gly Arg Arg Ala Ser
CGA-AAA-CGT-AGT-CAG-ATG-CTG-TTT-CAA-GGT-CGA-AGA-GCA-TCC-
GCT-TTT-GCA-TCA-GTC-TAC-GAC-AAA-GTT-CCA-GCT-TCT-CGT-AGG-

    stop BamHI
Gln
CAG-TAA-G
GTC-ATT-CCTAG
```

Fra-B-9 (134 bp) α-hANP gene with linker DNA

```
HindIII
 Lys Leu Glu Val Glu His Glu Phe Gly Met Gly Gly Glu Ala Lys
  AG-CTT-GAA-GTT-GAG-CAT-GAA-TTC-GGT-ATG-GGC-GGT-GAA-GCT-AAA-
        A-CTT-CAA-CTC-GTA-CTT-AAG-CCA-TAC-CCG-CCA-CTT-CGA-TTT-

Ser Leu Arg Arg Ser Ser Cys Phe Gly Gly Arg Met Asp Arg Ile
TCT-CTG-CGT-AGA-TCC-TCT-TGC-TTT-GGT-GGC-CGT-ATG-GAC-CGC-ATC-
AGA-GAC-GCA-TCT-AGG-AGA-ACG-AAA-CCA-CCG-GCA-TAC-CTG-GCG-TAG-

                                                  stop stop
BamHI
Gly Ala Gln Ser Gly Leu Gly Cys Asn Ser Phe Arg Tyr
GGT-GCT-CAG-TCC-GGT-CTG-GGC-TGT-AAC-TCT-TTC-CGT-TAC-TGA-TAG
CCA-CGA-GTC-AGG-CCA-GAC-CCG-ACA-TTG-AGA-AAG-GCA-ATG-ACT-ATC-CTA
```

The DNA fragment (Fra-A-7) useful in the construction of the present two-cistronic IGF-I expression vectors was prepared as follows.

Fra-B-4 was ligated into the large EcoRI-BamHI restriction fragment of plasmid pTrpEB7 to form plasmid pLHtrp encoding trp promoter I and LH. IGF-I-encoding gene was prepared for the construction of the present vectors in the following manner. Plasmid pBR322 was digested with restriction enzymes EcoRI and BamHI and the large restriction fragment was isolated, to which was then ligated the IGF-I encoding DNA (Fra-B-10) to form plasmid pSdm1. From the plasmid pSdm1, EcoRI-BamHI restriction fragment encoding IGF-I was isolated and to which fragment was ligated oligonucleotides m1 and m2 to form Fra-B-11. Fra-B-11 was ligated into the HindIII-BamHI restriction fragment of plasmid pLHtrp to form plasmid pLHSdMmtrp. The nucleotide and amino acids sequences of Fra-B-10 and Fra-B-11 are shown below. The illustrative construction protocol of plasmid pLHSdMmtrp is presented in Fig. 7.

<u>Fra-B-10</u> (224 bp) IGF-I gene

```
                    EcoRI      AvaII
                           Met Gly Pro Glu Thr Leu Cys Gly
                      AATTC-ATG-GGT-CCT-GAA-ACT-CTG-TGC-GGC-
                          G-TAC-CCA-GGA-CTT-TGA-GAC-ACG-CCG-

          10                                      20
Ala Glu Leu Val Asp Ala Leu Gln Phe Val Cys Gly Asp Arg
GCT-GAA-CTG-GTT-GAC-GCT-CTG-CAA-TTT-GTA-TGT-GGT-GAT-CGT-
CGA-CTT-GAC-CAA-CTG-CGA-GAC-GTT-AAA-CAT-ACA-CCA-CTA-GCA-

                                    30
Gly Phe Tyr Phe Asn Lys Pro Thr Gly Tyr Gly Ser Ser Ser
GGT-TTC-TAC-TTC-AAC-AAA-CCG-ACC-GGC-TAT-GGC-TCC-AGC-TCT-
CCA-AAG-ATG-AAG-TTG-TTT-GGC-TGG-CCG-ATA-CCG-AGG-TCG-AGA-

                40
Arg Arg Ala Pro Gln Thr Gly Ile Val Asp Glu Cys Cys Phe
CGT-CGC-GCA-CCG-CAG-ACT-GGT-ATC-GTA-GAC-GAA-TGC-TGT-TTT-
GCA-GCG-CGT-GGC-GTC-TGA-CCA-TAG-CAT-CTG-CTT-ACG-ACA-AAA-

 50                                     60
Arg Ser Cys Asp Leu Arg Arg Leu Glu Met Tyr Cys Ala Pro
CGT-TCT-TGC-GAT-CTC-CGC-CGT-CTG-GAA-ATG-TAC-TGT-GCT-CCA-
GCA-AGA-ACG-CTA-GAG-GCG-GCA-GAC-CTT-TAC-ATG-ACA-CGA-GGT-
```

```
                              70 stop stop BamHI
Leu Lys Pro Ala Lys Ser Ala
CTG-AAG-CCA-GCA-AAA-TCC-GCG-TGA-TAG-3'
GAC-TTC-GGT-CGT-TTT-AGG-CGC-ACT-ATC-CTAG-5'
```

Fra-B-11 (242 bp) IGF-I gene with linker DNA, same to Fra-A-4

```
HindIII                      EcoRI        AvaII
    Leu Glu Val Lys His Glu Phe Met Gly Pro Glu Thr Leu
  AG-CTT-GAA-GTA-AAA-CAT-GAA-TTC-ATG-GGT-CCT-GAA-ACT-CTG-
       A-CTT-CAT-TTT-GTA-CTT-AAG-TAC-CCA-GGA-CTT-TGA-GAC-

                  10
Cys Gly Ala Glu Leu Val Asp Ala Leu Gln Phe Val Cys Gly
TGC-GGC-GCT-GAA-CTG-GTT-GAC-GCT-CTG-CAA-TTT-GTA-TGT-GGT-
ACG-CCG-CGA-CTT-GAC-CAA-CTG-CGA-GAC-GTT-AAA-CAT-ACA-CCA-

 20                                     30
Asp Arg Gly Phe Tyr Phe Asn Lys Pro Thr Gly Tyr Gly Ser
GAT-CGT-GGT-TTC-TAC-TTC-AAC-AAA-CCG-ACC-GGC-TAT-GGC-TCC-
CTA-GCA-CCA-AAG-ATG-AAG-TTG-TTT-GGC-TGG-CCG-ATA-CCG-AGG-

                        40
Ser Ser Arg Arg Ala Pro Gln Thr Gly Ile Val Asp Glu Cys
AGC-TCT-CGT-CGC-GCA-CCG-CAG-ACT-GGT-ATC-GTA-GAC-GAA-TGC-
TCG-AGA-GCA-GCG-CGT-GGC-GTC-TGA-CCA-TAG-CAT-CTG-CTT-ACG-

          50                                        60
Cys Phe Arg Ser Cys Asp Leu Arg Arg Leu Glu Met Tyr Cys
TGT-TTT-CGT-TCT-TGC-GAT-CTC-CGC-CGT-CTG-GAA-ATG-TAC-TGT--
ACA-AAA-GCA-AGA-ACG-CTA-GAG-GCG-GCA-GAC-CTT-TAC-ATG-ACA-
```

```
                              70 stop stop BamHI
Ala Pro Leu Lys Pro Ala Lys Ser Ala
GCT-CCA-CTG-AAG-CCA-GCA-AAA-TCC-GCG-TGA-TAG-3'
CGA-GGT-GAC-TTC-GGT-CGT-TTT-AGG-CGC-ACT-ATC-CTAG-5'
```

Plasmid pLHSdMmtrp was digested with restriction enzymes HindIII and BamHI to obtain Fra-A-4 (same as Fra-B-11) containing a gene encoding IGF-I with linker DNA. The fragment was digested with AvaII to give Fra-A-5 containing a translational termination codon at 3′ terminus.

Fra-A-3 which comprises SD sequence and termination codon was constructed by ligating the ClaI restriction fragment of Fra-A-1 (i.e., Fra-A-2) to oligonucleotides CT5 and CT6. Fra-A-3 was ligated to Fra-A-5 at the AvaII restriction site to form Fra-A-6 which contained SD sequence, translational termination signal, translational initiation signal and IGF-I-encoding gene in series. Fra-A-6 was digested with HindIII restriction enzyme to obtain Fra-A-7. The nucleotide and amino acid sequences and the restriction sites of Fra-A-1, Fra-A-2, Fra-A-3, Fra-A-4. Fra-A-5, Fra-A-6 and Fra-A-7 are shown below. The illustrative synthesis protocol of Fra-A-7 is represented in Fig. 8.

<u>Fra-A-1</u> (46 bp) Linker DNA containing internal SD sequence

```
                                HindIII         ClaI  stop
Arg Cys Gln Tyr Arg Asp Leu Lys Leu Glu Glu Asn Arg    Met
 GT-TGC-CAG-TAC-CGC-GAC-CTG-AAG-CTT-GAG-GAG-AAT-CGA-TA-ATG-
          C-ATG-GCG-CTG-GAC-TTC-GAA-CTC-CTC-TTA-GCT-AT-TAC-

Ser Leu Arg
TCT
AGA-GAC-GC
```

Fra-A-2 (35 bp) Linker DNA containing internal SD sequence

```
                               HindIII             ClaI
Arg Cys Gln Tyr Arg Asp Leu Lys Leu Glu Glu Asn
 GT-TGC-CAG-TAC-CGC-GAC-CTG-AAG-CTT-GAG-GAG-AAT
           C-ATG-GCG-CTG-GAC-TTC-GAA-CTC-CTC-TTA-GC
```

Fra-A-3 (44 bp) Linker DNA containing internal SD sequence

```
                               HindIII             ClaI  stop  AvaII
Arg Cys Gln Tyr Arg Asp Leu Lys Leu Glu Glu Asn Arg   Met Gly
 GT-TGC-CAG-TAC-CGC-GAC-CTG-AAG-CTT-GAG-GAG-AAT-CGA-TA-ATG-G
           C-ATG-GCG-CTG-GAC-TTC-GAA-CTC-CTC-TTA-GCT-AT-TAC-CCA-G
```

Fra-A-4 (242 bp) IGF-I gene with linker DNA, same to Fra-B-11

```
                                HindIII
                                    Leu Glu Val Lys His-
                                 AG-CTT-GAA-GTA-AAA-CAT-
                                     A-CTT-CAT-TTT-GTA-
```

```
            AvaII
Glu Phe Met Gly Pro Glu Thr Leu Cys Gly Ala Glu Leu Val
GAA-TTC-ATG-GGT-CCT-GAA-ACT-CTG-TGC-GGC-GCT-GAA-CTG-GTT-
CTT-AAG-TAC-CCA-GGA-CTT-TGA-GAC-ACG-CCG-CGA-CTT-GAC-CAA-
```

```
Asp Ala Leu Gln Phe Val Cys Gly Asp Arg Gly Phe Tyr Phe
GAC-GCT-CTG-CAA-TTT-GTA-TGT-GGT-GAT-CGT-GGT-TTC-TAC-TTC-
CTG-CGA-GAC-GTT-AAA-CAT-ACA-CCA-CTA-GCA-CCA-AAG-ATG-AAG-
```

```
Asn Lys Pro Thr Gly Tyr Gly Ser Ser Ser Arg Arg Ala Pro
AAC-AAA-CCG-ACC-GGC-TAT-GGC-TCC-AGC-TCT-CGT-CGC-GCA-CCG-
TTG-TTT-GGC-TGG-CCG-ATA-CCG-AGG-TCG-AGA-GCA-GCG-CGT-GGC-

Gln Thr Gly Ile Val Asp Glu Cys Cys Phe Arg Ser Cys Asp
CAG-ACT-GGT-ATC-GTA-GAC-GAA-TGC-TGT-TTT-CGT-TCT-TGC-GAT-
GTC-TGA-CCA-TAG-CAT-CTG-CTT-ACG-ACA-AAA-GCA-AGA-ACG-CTA-

Leu Arg Arg Leu Glu Met Tyr Cys Ala Pro Leu Lys Pro Ala
CTC-CGC-CGT-CTG-GAA-ATG-TAC-TGT-GCT-CCA-CTG-AAG-CCA-GCA-
GAG-GCG-GCA-GAC-CTT-TAC-ATG-ACA-CGA-GGT-GAC-TTC-GGT-CGT-

            stop BamHI
Lys Ser Ala
AAA-TCC-GCG-TGA-TAG
TTT-AGG-CGC-ACT-ATC-CTAG
```

<u>Fra-A-5</u> (215 bp)

```
    AvaII
        Pro Glu Thr Leu Cys Gly Ala Glu Leu Val
      GT-CCT-GAA-ACT-CTG-TGC-GGC-GCT-GAA-CTG-GTT-
           GA-CTT-TGA-GAC-ACG-CCG-CGA-CTT-GAC-CAA-

Asp Ala Leu Gln Phe Val Cys Gly Asp Arg Gly Phe Tyr Phe
GAC-GCT-CTG-CAA-TTT-GTA-TGT-GGT-GAT-CGT-GGT-TTC-TAC-TTC-
CTG-CGA-GAC-GTT-AAA-CAT-ACA-CCA-CTA-GCA-CCA-AAG-ATG-AAG-

Asn Lys Pro Thr Gly Tyr Gly Ser Ser Ser Arg Arg Ala Pro
AAC-AAA-CCG-ACC-GGC-TAT-GGC-TCC-AGC-TCT-CGT-CGC-GCA-CCG-
TTG-TTT-GGC-TGG-CCG-ATA-CCG-AGG-TCG-AGA-GCA-GCG-CGT-GGC-
```

```
Gln Thr Gly Ile Val Asp Glu Cys Cys Phe Arg Ser Cys Asp
CAG-ACT-GGT-ATC-GTA-GAC-GAA-TGC-TGT-TTT-CGT-TCT-TGC-GAT-
GTC-TGA-CCA-TAG-CAT-CTG-CTT-ACG-ACA-AAA-GCA-AGA-ACG-CTA-

Leu Arg Arg Leu Glu Met Tyr Cys Ala Pro Leu Lys Pro Ala
CTC-CGC-CGT-CTG-GAA-ATG-TAC-TGT-GCT-CCA-CTG-AAG-CCA-GCA-
GAG-GCG-GCA-GAC-CTT-TAC-ATG-ACA-CGA-GGT-GAC-TTC-GGT-CGT-

            stop BamHI
Lys Ser Ala
AAA-TCC-GCG-TGA-TAG
TTT-AGG-CGC-ACT-ATC-CTAG
```

<u>Fra-A-6</u> (259 bp)

```
                           HindIII          ClaI  stop
Arg Cys Gln Tyr Arg Asp Leu Lys Leu Glu Glu Asn Arg
 GT-TGC-CAG-TAC-CGC-GAC-CTG-AAG-CTT-GAG-GAG-AAT-CGA-TA-
         C-ATG-GCG-CTG-GAC-TTC-GAA-CTC-CTC-TTA-GCT-AT-

   AvaII
Met Gly Pro Glu Thr Leu Cys Gly Ala Glu Leu Val
ATG-GGT-CCT-GAA-ACT-CTG-TGC-GGC-GCT-GAA-CTG-GTT-
TAC-CCA-GGA-CTT-TGA-GAC-ACG-CCG-CGA-CTT-GAC-CAA-

Asp Ala Leu Gln Phe Val Cys Gly Asp Arg Gly Phe Tyr Phe
GAC-GCT-CTG-CAA-TTT-GTA-TGT-GGT-GAT-CGT-GGT-TTC-TAC-TTC-
CTG-CGA-GAC-GTT-AAA-CAT-ACA-CCA-CTA-GCA-CCA-AAG-ATG-AAG-

Asn Lys Pro Thr Gly Tyr Gly Ser Ser Ser Arg Arg Ala Pro
AAC-AAA-CCG-ACC-GGC-TAT-GGC-TCC-AGC-TCT-CGT-CGC-GCA-CCG-
TTG-TTT-GGC-TGG-CCG-ATA-CCG-AGG-TCG-AGA-GCA-GCG-CGT-GGC-
```

```
Gln Thr Gly Ile Val Asp Glu Cys Cys Phe Arg Ser Cys Asp
CAG-ACT-GGT-ATC-GTA-GAC-GAA-TGC-TGT-TTT-CGT-TCT-TGC-GAT-
GTC-TGA-CCA-TAG-CAT-CTG-CTT-ACG-ACA-AAA-GCA-AGA-ACG-CTA-

Leu Arg Arg Leu Glu Met Tyr Cys Ala Pro Leu Lys Pro Ala
CTC-CGC-CGT-CTG-GAA-ATG-TAC-TGT-GCT-CCA-CTG-AAG-CCA-GCA-
GAG-GCG-GCA-GAC-CTT-TAC-ATG-ACA-CGA-GGT-GAC-TTC-GGT-CGT-

            stop stop BamHI
Lys Ser Ala
AAA-TCC-GCG-TGA-TAG
TTT-AGG-CGC-ACT-ATC-CTAG
```

Fra-A-7 (238 bp)

```
                         HindIII            ClaI  stop
                             Lys Glu Glu Asn Arg
                          AG-CTT-GAG-GAG-AAT-CGA-TA-
                              A-CTC-CTC-TTA-GCT-AT-

     AvaII
Met Gly Pro Glu Thr Leu Cys Gly Ala Glu Leu Val
ATG-GGT-CCT-GAA-ACT-CTG-TGC-GGC-GCT-GAA-CTG-GTT-
TAC-CCA-GGA-CTT-TGA-GAC-ACG-CCG-CGA-CTT-GAC-CAA-

Asp Ala Leu Gln Phe Val Cys Gly Asp Arg Gly Phe Tyr Phe
GAC-GCT-CTG-CAA-TTT-GTA-TGT-GGT-GAT-CGT-GGT-TTC-TAC-TTC-
CTG-CGA-GAC-GTT-AAA-CAT-ACA-CCA-CTA-GCA-CCA-AAG-ATG-AAG-

Asn Lys Pro Thr Gly Tyr Gly Ser Ser Ser Arg Arg Ala Pro
AAC-AAA-CCG-ACC-GGC-TAT-GGC-TCC-AGC-TCT-CGT-CGC-GCA-CCG-
TTG-TTT-GGC-TGG-CCG-ATA-CCG-AGG-TCG-AGA-GCA-GCG-CGT-GGC-
```

```
Gln Thr Gly Ile Val Asp Glu Cys Cys Phe Arg Ser Cys Asp
CAG-ACT-GGT-ATC-GTA-GAC-GAA-TGC-TGT-TTT-CGT-TCT-TGC-GAT-
GTC-TGA-CCA-TAG-CAT-CTG-CTT-ACG-ACA-AAA-GCA-AGA-ACG-CTA-

Leu Arg Arg Leu Glu Met Tyr Cys Ala Pro Leu Lys Pro Ala
CTC-CGC-CGT-CTG-GAA-ATG-TAC-TGT-GCT-CCA-CTG-AAG-CCA-GCA-
GAG-GCG-GCA-GAC-CTT-TAC-ATG-ACA-CGA-GGT-GAC-TTC-GGT-CGT-

            stop stop BamHI
Lys Ser Ala
AAA-TCC-GCG-TGA-TAG
TTT-AGG-CGC-ACT-ATC-CTAG
```

Fra-A-7 was then ligated into the HindIII-BamHI restriction fragment of α-hANP expression vector pCLaHtrpSd to replace the α-hANP-encoding gene with IGF-I-encoding gene to form the plasmid of the invention, pCE-SMtrp. The illustrative construction protocol is presented in Fig. 9.

Another IGF-I expression vector pCE-SM3t was constructed as follows.

The DNA sequence encoding Trp promoter III (Fra-B-3) was ligated into the EcoRI-ClaI restriction fragment of plasmid pBR322 to obtain plasmid pBR322trpSs containing $Amp^R$ and $Tet^R$. The ClaI-BamHI restriction fragment of plasmid pCLaHtrpSd (Fra-C-2 encoding Cd-LH and α-hANP) was ligated into the large ClaI-BamHI restriction fragment of plasmid pBR322trpSs to form plasmid pCLaHtrp-2. The nucleotide and amino acid sequences of Fra-C-2 are shown below. The illustrative construction protocol of plasmid pCLaHtrp-2 is presented in Fig. 10.

Fra-C-2 (406 bp)

```
   ClaI      Met Phe Tyr Phe Asn Lys Pro Thr Gly
     CGATAAA-ATG-TTC-TAC-TTC-AAC-AAA-CCG-ACC-GGC-
       TATTT-TAC-AAG-ATG-AAG-TTG-TTT-GGC-TGG-CCG-

Tyr Gly Ser Ser Ser Arg Arg Ala Pro Gln Thr Gly Ile Val Asp
TAT-GGC-TCC-AGC-TCT-CGT-CGC-GCA-CCG-CAG-ACT-GGT-ATC-GTA-GAC-
ATA-CCG-AGG-TCG-AGA-GCA-GCG-CGT-GGC-GTC-TGA-CCA-TAG-CAT-CTG-

                EcoRI
Glu Gly Gly Asp Glu Phe Met Cys Tyr Cys Gln Asp Pro Tyr
GAG-GGT-GGC-GAT-GAA-TTC-ATG-TGT-TAC-TGC-CAG-GAC-CCA-TAT-
CTC-CCA-CCG-CTA-CTT-AAG-TAC-ACA-ATG-ACG-GTC-CTG-GGT-ATA-

        10                                      20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-

                                    30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

                    40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-
```

```
 50                             HindIII   60
Ser Gln Ile Val Ser Phe Tyr Phe Lys Leu Glu Val Glu
AGC-CAA-ATT-GTC-TCC-TTT-TAC-TTC-AAG-CTT-GAA-GTT-GAG-
TCG-GTT-TAA-CAG-AGG-AAA-ATG-AAG-TTC-GAA-CTT-CAA-CTC-

His Glu Phe Gly Met Gly Gly Glu Ala Lys
CAT-GAA-TTC-GGT-ATG-GGC-GGT-GAA-GCT-AAA-
GTA-CTT-AAG-CCA-TAC-CCG-CCA-CTT-CGA-TTT-

Ser Leu Arg Arg Ser Ser Cys Phe Gly Gly Arg Met Asp Arg
TCT-CTG-CGT-AGA-TCC-TCT-TGC-TTT-GGT-GGC-CGT-ATG-GAC-CGC-
AGA-GAC-GCA-TCT-AGG-AGA-ACG-AAA-CCA-CCG-GCA-TAC-CTG-GCG-

Ile Gly Ala Gln Ser Gly Leu Gly Cys Asn Ser Phe Arg Tyr
ATC-GGT-GCT-CAG-TCC-GGT-CTG-GGC-TGT-AAC-TCT-TTC-CGT-TAC-
TAG-CCA-CGA-GTC-AGG-CCA-GAC-CCG-ACA-TTG-AGA-AAG-GCA-ATG-

stop stop BamHI

TGA-TAG
ACT-ATC-CTA
```

A synthetic fd phage terminator-encoding gene (Fra-C-1) was ligated into the BamHI-SalI restriction fragment of plasmid pBR322 to form plasmid pter21. The plasmid pter21 was digested with restriction enzymes PstI and BamHI to obtain a DNA fragment containing the fd phage terminator-encoding DNA, and the latter was ligated to the large BamHI-PstI restriction fragment of plasmid pCLaHtrp-2 to form plasmid pCLaHtrp3t. The nucleotide and amino acid sequences of Fra-C-1 are shown below. The illustrative construction protocol of plasmid pCLaHtrp3t is presented in Fig. 11.

<u>Fra-C-1</u> (47 bp) Synthetic fd phage terminator gene

```
GATCCTCGAGATCAATTAAAGGCTCCTTTTGGAGCCTTTTTTTTTTG
    GAGCTCTAGTTAATTTCCGAGGAAAACCTCGGAAAAAAAAAACAGCT
```

The previously described Fra-A-7 was ligated into the BamHI-HindIII restriction fragment of plasmid

pCLaHtrp3t to form two-cistronic expression vector pCE-SM3t which encodes trp promoter III, Cd-LH, IGF-I and fd phage terminator. The illustrative construction protocol is presented in Fig. 12.

DNA fragment used for the construction of present expression vectors was conventionally prepared by annealing and ligating the oligonucleotides which were prepared in accordance with the method of Ito, H., Ike, Y., Ikuta, S. and Itakura, K., Nucleic Acids Research, 10, 1, 755(1982).

Although expression vectors of the invention can be applied to a wide range of E. coli host cells, E. coli K and E. coli B strains are most preferred as host cells. E. coli MM 294 is especially preferable. The present expression vector can be transformed into E. coli host cell using conventional methods such as Kushner's method. When the transformant is cultured under suitable conditions for growth, genes encoding Cd-LH and Met-IGF-I are expressed individually to produce each corresponding peptide. IGF-I is safely maintained inside of the host cell by virture of the protective action of Cd-LH. Preferred cultivation methods are well known. E. coli is usually grown under aerobic culture conditions (e.g., shaking culture or submerged liquid culture) in nutrient medium containing assimilable carbon and nitrogen sources. Preferred carbon sources in a culture medium include, for example, carbohydrates such as glucose, fructorse, sucrose, glycerol, and starch. Other carbon sources may include xylose, galactose, maltose, dextrin, and lactose.

Preferred nitrogen sources include, for example, yeast extract, peptone, gluten meal, cottonseed meal, soybean meal, corn steep liquor, dried yeast, and malt of wheat. Inorganic and organic nitrogen compounds such as ammonium nitrate, ammonium sulfate, ammonium phosphate, urea, and amino acids can be also included in the culture medium.

It is not necessarily required to use these carbon and nitrogen sources in a purified form, since relatively impure materials often contain a trace amounts of essential elements required for the growth and considerable amounts of inorganic nutrients, and therefore, they are also suitable for cultivation. If desired, inorganic salts such as calcium bicarbonate, sodium or potassium phosphate, sodium or potassium chloride, magnesium or cupric salts may be added into the culture medium.

Mixing of the culture medium can be accomplished using mechanical stirrers such as propeller or various devices with pump, or by rotating or shaking the fermenter, or aerating sterilized air into the culture medium. Fermentation are usually conducted at temperature ranging from about 20 to 42°C, preferably about 35 - 38°C, for several hours to 50 hours.

The expressed Met-IGF-I is retained inside of transformants together with Cd-LH, probably in an associated form. The associated Met-IGF-I can be easily recovered from the culture medium using conventional methods. Generally, it can be conducted as follows. Transformed cells are collected by filtration or centrifugation and lysed by vaccum concentration or ultrasonification. From the resultant lysate, the desired Met-IGF-I can be isolated using HPLC, lyophylization, pH adjustments, adsorption on appropriate resins such as anionic, cathionic, or nonionic resins, adsorbents (e.g., carbon, silic acid, silica gel, cellulose, or alumina), gel filteration, recrystallization from suitable solvents or combination thereof. In the illustrative example, the lysate is dialysed against 1N acetic acid to separate soluble Met-IGF-I from insoluble Cd-LH, and the dialyzed solution is subjected to purification process.

The present invention provides for an efficient means for producing a large amount of Met-IGF-I in purified form as compared with conventional methods. Met-IGF-I provided by the present invention is useful for the treatment of various diseases caused by disorder of proliferation or growth of bone cells, or other diseases such as osteoporosis, deabetes, wound or ulcer. Met-IGF-I can be formulated into a pharmaceutical composition suitable for oral or parenteral administration in accordance with conventional methods known to the art.

In the accompanying drawings:

Figure 1 shows the nucleotide and amino acid sequence of Met-IGF-I.

Figure 2 (1) is a restriction site and functional map of plasmid pCE-SMtrp, and Fig. 2 (2) is a restriction site and functional map of plasmid pCE-SM3t.

Figure 3 is a schematic illustration of the construction protocol for plasmid pTrpEB7.

Figure 4 is a schematic illustration of the construction protocol for plasmid p322dtrpS.

Figure 5 is a schematic illustration of the construction protocol for plasmid pCd$\gamma$.

Figure 6 is a schematic illustration of the construction protocol for plasmid pCLaHtrpSd.

Figure 7 is a schematic illustration of the construction protocol for plasmid pLHSdmMtrp.

Figure 8 is a schematic illustration of the synthesis protocol for DNA fragment (Fra-A-7) used for the construction of two-cistronic expression vector.

Figure 9 is a schematic illustration of the construction protocol for plasmid pCE-SMtrp.

Figure 10 is a schematic illustration of the construction protocol for plasmid pCLaHtrp-2.

Figure 11 is a schematic illustration of the construction protocol for plasmid pCLaHtrp3t.

Figure 12 is a schematic illustration of the construction protocol for plasmid pCE-SM3t.

Figure 13 is a schematic illustration of the synthesis protocol for DNA fragments encoding LH and RH (Fra-B-4 and Fra-B-5).
Figure 14 is a schematic illustration of the synthesis protocol for Fra-B-1.
Figure 15 is a schematic illustration of the synthesis protocol for Fra-B-3.
Figure 16 is a schematic illustration of the synthesis protocol for Fra-B-7.
Figure 17 is a schematic illustration of the synthesis protocol for Fra-B-9.
Figure 18 is a schematic illustration of the synthesis protocol for Fra-B-10.

The following preparations and examples further illustrate the invention.

DNA oligomers used for the synthesis of DNA fragments in the following preparations and examples were prepared in substantial accordance with the procedure of Ito, S. et al., (1982), Nucleic Acids Research, 10 175. The DNA fragments can also be synthesized by the modified phosphotriester method using protected deoxyribonucleotide building blocks.

Unless otherwise stated, the isolation of a desired DNA fragment from a plasmid was accomplished by digesting said plasmid with restriction enzyme(s) and separating the desired fragment from the digestion mixture using polyacrylamide gel electrophoresis.

## Preparation 1

### Preparation of Gene Encoding LH-RH (Fra-B-6)

For the preparation of Fra-B-4 and Fra-B-5 (oligonucleotides for IFN LH gene and IFN RH gene), the 63 oligonucleotides were synthesized using conventional methods.

(1) HOApApTpTpCpApTpGpTpGpTpTOH (a1)

(2) HOApCpTpGpCpCpApGpGpApCpCpCpApTOH (a2)

(3) HOApTpGpTpApApApApGpApApGpCpApGOH (a3)

(4) HOTpGpGpCpApGpTpApApCpApCpApTpGOH (a4)

(5) HOTpTpTpApCpApTpApTpGpGpGpTpCpCOH (a5)

(6) HOApApGpGpTpTpTpTpCpTpGpCpTpTpCpTOH (a6)

(7) HOApApApApCpCpTpTpApApGpApApApTpAOH (b1)

(8) HOCpTpTpTpApApTpGpCpApGpGpTpCpAOH (b2)

(9) HOTpTpCpApGpApTpGpTpApGpCpGpGpAOH (b3)

(10) HOApTpTpApApApGpTpApTpTpTpCpTpTOH (b4)

(11) HOApTpCpTpGpApApTpGpApCpCpTpGpCOH (b5)

(12) HOTpTpCpCpApTpTpApTpCpCpGpCpTpApCOH (b6)

(13) HOTpApApTpGpGpApApCpTpCpTpTpTpTpCOH (c1)

(14) HOTpTpApGpGpCpApTpTpTpTpGpApApGOH (c2)

(15) HOApApTpTpGpGpApApApGpApGpGpApGOH (c3)

(16) HOTpGpCpCpTpApApGpApApApApGpApGOH (c4)

(17) HOTpCpCpApApTpTpCpTpTpCpApApApAOH (c5)

(18) HOCpTpGpTpCpApCpTpCpTpCpCpTpCpTpTOH (c6)

(19) HOApGpTpGpApCpApGpApApApApApTpAOH (d1)

(20) HOApTpGpCpApGpApGpCpCpApApApTpTOH (d2)

(21) HOGpTpCpTpCpCpTpTpTpTpApCpTpTOH (d3)

(22) HOCpTpCpTpGpCpApTpTpApTpTpTpTpTOH (d4)

(23) HOApGpGpApGpApCpApApTpTpTpGpGOH (d5)

(24) HOApApApGpCpTpTpGpApApGpTpApApAOH (d6)

(25) HOCpApApGpCpTpTpTpTpCpApApApApAOH (e1)

(26) HOCpTpTpTpApApGpGpApTpGpApCpCpAOH (e2)

(27) HOGpApGpCpApTpCpCpApApApApGpApGOH (e3)

(28) HOCpCpTpTpApApApGpTpTpTpTpTpGpAOH (e4)

(29) HOGpGpApTpGpCpTpCpTpGpGpTpCpApTOH (e5)

(30) HOTpCpTpCpCpApCpApCpTpCpTpTpTpTOH (e6)

(31) HOTpGpTpGpGpApGpApCpCpApTpCpApAOH (f1)

(32) HOGpGpApApGpApCpApTpGpApApTpGpTOH (f2)

(33) HOCpApApGpTpTpTpTpTpCpApApTpApGOH (f3)

(34) HOTpGpTpCpTpTpCpCpTpTpGpApTpGpGOH (f4)

(35) HOApApApApCpTpTpGpApCpApTpTpCpAOH (f5)

(36) HOTpTpTpTpGpTpTpGpCpTpApTpTpGpAOH (f6)

(37) HOCpApApCpApApApApApGpApApApCpGOH (g1)

(38) HOTpGpApTpGpApCpTpTpCpGpApApApAOH (g2)

(39) HOGpCpTpGpApCpTpApApTpTpApTpTpCOH (g3)

(40) HOApGpTpCpApTpCpApCpGpTpTpTpCpTOH (g4)

(41) HOTpApGpTpCpApGpCpTpTpTpTpCpGpAOH (g5)

(42) HOCpApGpTpTpApCpCpGpApApTpApApTOH (g6)

(43) HOGpGpTpApApCpTpGpApCpTpTpGpApAOH (h1)

(44) HOTpGpTpCpCpApApCpGpCpApApApGpCOH (h2)

(45) HOApApTpApCpApTpGpApApCpTpCpApTpCOH (h3)

(46) HOGpTpTpGpGpApCpApTpTpCpApApGpTOH (h4)

(47) HOCpApTpGpTpApTpTpGpCpTpTpTpGpCOH (h5)

(48) HOApTpCpApCpTpTpGpGpApTpGpApGpTpTOH (h6)

(49) HOCpApApGpTpGpApTpGpGpCpTpGpApAOH (i1)

(50) HOCpTpGpTpCpGpCpCpApGpCpApGpCpTOH (i2)

(51) HOApApApApCpApGpGpGpApApGpCpGpAOH (i3)

(52) HOGpGpCpGpApCpApGpTpTpCpApGpCpCOH (i4)

(53) HOCpCpTpGpTpTpTpTpApGpCpTpGpCpTOH (i5)

(54) HOCpTpApCpGpTpTpTpTpCpGpCpTpTpCOH (i6)

(55) HOApApApCpGpTpApGpTpCpApGpApTpGpCOH (j1)

(56) HOTpGpTpTpTpCpApApGpGpTpCpGpApAOH (j2)

(57) HOGpApGpCpApTpCpCpCpApGpTpApApGOH (j3)

(58) HOTpGpApApApCpApGpCpApTpCpTpGpAOH (j4)

(59) HOGpApTpGpCpTpCpTpTpCpGpApCpCpTOH (j5)

(60) HOGpApTpCpCpTpTpApCpTpGpGOH (j6)

(61) HOTpGpTpGpTpApApTpGpApTpApGOH (l1)

(62) HOTpApCpApCpApCpTpCpTpTpTpTOH (l2)

(63) HOGpApTpCpCpTpApTpCpApTOH (l3)

(wherein, Ap is deoxyadenyl, Gp is deoxyguanyl, Cp is deoxycitidyl and Tp is thymidyl).

A. Fra-B-4 (DNA Fragment Encoding LH)

A.1. Ligation of Synthetic Oligonucleotides

Each oligonucleotide (A2 - L2) (each 0.4 nM) was phosphorylated with T4 polynucleotide kinase (BRL; 2.5U) in 40µg/ml of ligation buffer (50mM Tris-HCl, pH 7.6, 20 mM DTT, 50µg/ml BSA, 1mM spermidine, 10mM $MgCl_2$ and 2 mM ATP) at 37° C for 3 hours. The reaction mixture was incubated at 100° C for 5 minutes to inactive the enzyme. Phosphorylated oligonucleotides were ligated to two oligonucleotides (A1

and L3) in accordance with the procedure illustrated in Fig. 13 (1), to form a DNA fragment encoding LH (236 bp). The ligation reaction was conducted in a reaction mixture containing 1µl of 50mM ATP at 16°C for 5 hours. Each ligation product was separated on 2-16% gradient PAGE (polyacrylamide gel electrophoresis) in Tris-EDTA buffer and visualized by ethidium bromide.

A.2. Cloning of LH-encoding gene

Plasmid pBR322 was digested with BamHI and EcoRI and the reaction was terminated by heating at 65°C for 5 minutes. The large DNA fragment was isolated on 0.5% agarose gel electrophoresis and ligated to DNA fragment encoding LH in the presence of T4 DNA ligase at 12°C for 18 hours. The ligated DNA constituted the desired plasmid pLH107. The resultant ligation mixture was used to transform E. coli HB101 in substantial accordance with the procedure of Kushner's method (Maniatis, T. et al., Molecular Cloning, pp 252, 1982, Cold Spring Harber Laboratories) and the transformants were selected on agar plate containing 25µg/ml tetracycline. Ampicillin resistant and tetracycline sensitive clone was selected. The plasmid was isolated from the clone and digested with EcoRI and BamHI, and confirmed the presence of the desired 236 bp LH-encoding gene on the basis of the size of the fragment compared with an appropriate size marker.

The plasmid pLH107 isolated from E. coli HB101 transformant was characterized by restriction analysis to contain the DNA fragment encoding LH.

B. Fra-B-5 (DNA Sequence Encoding RH)

B.1. Ligation of Synthetic Oligonucleotides

Each oligonucleotides (D3 - J5) (each 0.4mM) was phosphorylated in accordance with the procedure described in A.1., and ligated to an oligonucleotide (J6) in accordance with the procedure shown in Fig. 13 (2) to yield a DNA fragment encoding RH (270 bp). The ligation mixture was purified on PAGE to give the 270 bp RH-encoding DNA, which was used for the cloning of LH-RH-encoding DNA.

The synthesis protocol for Fra-B-4 and Fra-B-5 are shown in Figure 13, (1) and (2).

B.2 Cloning of LH-RH-encoding gene

Fra-B-5 was ligated into the large HindIII-BamHI restriction fragment of plasmid pLH 107 in the presence of T4 DNA ligase. The ligation mixture was used to transform E. coli HB 101 and plasmid pγF-3 was isolated from the transformants. The 450 bp EcoRI-BamHI restriction fragment encoding LH-RH (Fra-B-6) was obtained from the plasmid.

Preparation 2

Synthesis and Cloning of DNA Fragment Encoding Trp Promoter II (Fra-B-1)

Fra-B-1 was synthesized in substantial accordance with the teaching of Preparation 1, and ligated into the EcoRI-BamHI restriction fragment of pBR322, and the ligation mixture was used to transform E. coli HB101. The $Amp^R$ and $Tet^S$ transformant was selected, and the plasmid obtained therefrom was digested with HpaI to identify a 4.1 kbp band, which was then digested with BamHI, and a 90 bp band was confirmed on PAGE. A 56 bp EcoRI-BamHI restriction fragment of said 90bp DNA fragment was confirmed by PAGE using a size marker. The synthesis protocol for Fra-B-1 is presented in Figure 14. The oligonucleotides used for the synthesis of Fra-B-1 is as follows.

Fra-B-1 Oligonucleotides for synthetic trp promoter II gene

(1) HOApApTpTpTpGpCpCpGpApCpAOH (A)

(2) HOCpGpTpTpApTpGpApTpGpTpCpGpGpCpAOH (B)

(3) HOTpCpApTpApApCpGpGpTpTpCpTpGpGpCOH (C)

(4) HOGpApApTpApTpTpTpGpCpCpApGpApApCOH (D)

(5) HOApApApTpApTpTpCpTpGpApApApTpGpAOH (E)

(6) HOTpCpApApCpApGpCpTpCpApTpTpTpCpAOH (F)

(7) HOGpCpTpGpTpTpGpApCpApApTpTpApApTOH (G)

(8) HOGpTpTpCpGpApTpGpApTpTpApApTpTpGOH (H)

(9) HOCpApTpCpGpApApCpTpApGpTpTpApApCOH (I)

(10) HOGpCpGpTpApCpTpApGpTpTpApApCpTpAOH (J)

(11) HOTpApGpTpApCpGpCpApApGpTpTpCpApCOH (K)

(12) HOCpTpTpTpTpTpApCpGpTpGpApApCpTpTOH (L)

(13) HOGpTpApApApApApGpGpGpTpApTpCpGOH (M)

(14) HOApApTpTpCpGpApTpApCpCOH (N)

Preparation 3

Construction of Plasmid p322dtrpS Encoding Trip Promoters I and III

A. Construction of Plasmid pBR322trp

Five hundreds nanogram of 375 bp EcoRI-BamHI restriction fragment (Fra-B-2) was isolated from 9µg of plasmid pBR322 and the fragment (100ng) was ligated into 200µg of 4094 bp EcoRI-BamHI restriction fragment of plasmid pTrpEB7 (prepared in Preparation 2) in 20µl of ligation buffer containing 1mM ATP and T4 DNA ligase (360 units, Takara Syuzo, Inc.) at 15°C overnight. The ligation mixture was used to transform E. coli HB101, and $Tet^R$ transformant was selected on agar plate containing 25 µg/ml tetracycline. Plasmid pBR322trp was isolated from the transformant and the presence of the gene encoding trp promoter

I was confirmed by EcoRI-BamHI and HpaI digestion, and subsequent 7.5% PAGE and 0.8% agarose gel electrophoresis.

B. Preparation of DNA Fragment Encoding Trp Promoter III (Fra-B-3)

For the preparation of Fra-B-3, following oligonucleotides were synthesized.

Fra-B-3 Oligonucleotides for synthetic trp promoter III gene

(1) HOApApTpTpTpGpCpCpGpApCpAOH (A)

(2) HOCpGpTpTpApTpGpApTpGpTpCpGpGpCpAOH (B)

(3) HOTpCpApTpApApCpGpGpTpTpCpTpGpGpCOH (C)

(4) HOGpApApTpApTpTpTpGpCpCpApGpApApCOH (D)

(5) HOApApApTpApTpTpCpTpGpApApApTpGpAOH (E)

(6) HOTpCpApApCpApGpCpTpCpApTpTpTpCpAOH (F)

(7) HOGpCpTpGpTpTpGpApCpApApTpTpApApTOH (G)

(8) HOGpTpTpCpGpApTpGpApTpTpApApTpTpGOH (H)

(9) HOCpApTpCpGpApApCpTpApGpTpTpApApCOH (I)

(10) HOGpCpGpTpApCpTpApGpTpTpApApCpTpAOH (J)

(11) HOTpApGpTpApCpGpCpApApGpTpTpCpApCOH (K)

(12) HOCpTpTpTpTpTpApCpGpTpGpApApCpTpTOH (L)

(13) HOGpTpApApApApApGpGpGpTpApTOH (M')

(14) HOCpGpApTpApCpCOH(N')

Each of oligonucleotides (B-M') (0.2nM) of blocks I', II', and III' (Fig. 15) was phosphorylated with T4 polynucleotide kinase (2.5U, BRL) in 70 µl of ligation buffer at 37°C for 1 hour. To the reaction mixture of each block were added 300U of T4 DNA ligase and 2µl of 20mM ATP, and the mixture was incubated at 15°C for 30 minutes. The reaction was terminated by heating at 65°C for 10 minutes. The ligation mixtures of each block were combined and mixed with unphosphorylated oligonucleotides A and N' in the presence

of 360U of T4 DNA ligase and 2µl of 20 mM ATP. After incubation of the mixture at 15°C for 1 hour, the final ligation product was purified on 2-16% gradient polyacrylamide gel to give 105 bp trp promoter III-encoding DNA (Fra-B-3).

C. Construction of Plasmid p322dtrpS Encoding Trp Promoter I and III

Fra-B-3 was ligated into 4446 bp EcoRI-ClaI restriction fragment of plasmid pBR322trp. The resulting ligation mixture was used to transform E. coli HB101 and ampicillin-resistant and tetracycline-resistant transformants were selected. Plasmid p322 dtrpS was isolated from the transformant and the presence of ClaI-BamHI (352 bp), HpaI (107 bp), and AatII-ClaI (287 bp) restriction fragments were confirmed by restriction analysis. The schematic construction protocol is presented in Figure 4.

Preparation 4

Construction of Expression Vector pCdγ

A. Construction of LH-RH Expression Vector pγtrp

LH-RH gene (Fra-B-6, prepared in Preparation 1 B.2) was ligated into the 4.1 kbp EcoRI-BamHI restriction fragment of plasmid pTrpEB7 (prepared in Preparation 2). The ligation mixture was used to transform E. coli HB101 and $Amp^R$ and $Tet^S$ transformants were selected. Plasmid pγtrp was isolated from the transformants and the presence of 450 bp EcoRI-BamHI restriction fragment (Fra-B-6) was confirmed on 7.5% PAGE.

B. Construction of Plasmid pCdγ

B.1 Preparation of DNA Fragment Encoding Part of Trp Promoter III and Cd (Fra-B-7)

For the construction of Fra-B-7, the following 18 oligonucleotides were synthesized.

Fra-B-7 Oligonucleotides for peptide Cd gene with a part of DNA fragment of synthetic trp promoter III gene

(1) HOApApCpTpApGpTpApCpGpCOH (Np1)

(2) HOApApCpTpTpGpCpGpTpApCpTpApGpTpTOH (Np4)

(3) HOApApGpTpTpCpApCpGpTpApApApApApGOH (Np2)

(4) HOApTpApCpCpCpTpTpTpTpTpApCpGpTpGOH (Np5)

(5) HOGpGpTpApTpCpGpApTpApApApApTpGOH (Np3)

(6) HOGpTpApGpApApCpApTpTpTpTpApTpCpGOH (Np6)

(7) HOTpTpCpTpApCpTpTpCpApApCpApApAOH (Cd1)

(8) HOGpGpTpCpGpGpTpTpTpGpTpTpGpApAOH (Cd2)

(9) HOCpCpGpApCpCpGpGpCpTpApTpGOH (Cd3)

(10) HOGpCpTpGpGpApGpCpCpApTpApGpCpCOH (G'2)

(11) HOGpCpTpCpCpApGpCpTpCpTpCpGpTpCOH (H'1)

(12) HOCpGpGpTpGpCpGpCpGpApCpGpApGpAOH (H'2)

(13) HOGpCpGpCpApCpCpGpCpApGpApCpTpGOH (I'1)

(14) HOGpApTpApCpCpApGpTpCpTpGOH (Cd4)

(15) HOGpTpApTpCpGpTpApGpApCpGOH (Cd5)

(16) HOApCpCpCpTpCpGpTpCpTpApCOH (Cd6)

(17) HOApGpGpGpTpGpGpCpGpApTpGOH (Cd7)

(18) HOApApTpTpCpApTpCpGpCpCOH (Cd8)

Each of oligonucleotides (Np1-Cd8) (0.2nM) of blocks I″, II″, and III″ (Fig. 16) were phosphorylated with T4 nucleotide kinase (2.5U, BRL) in 60μl of ligation buffer at 37°C for 1 hour. To the reaction mixture of each block were added 360U of T4 DNA ligase and 2μl of 20 mM ATP, and the mixture was incubated at 15 °C for 1 hour. The reaction mixtures of each blocks were combined and incubated in the presence of 360U of T4 DNA ligase and 2μl of 20mM ATP overnight at 15°C, and then 10 minutes at 80°C. To the reaction mixture were added 20μl of 500mM NaCl and 20U of EcoRI. After incubation of the mixture at 37°C for 2 hours, the final ligation product was purified on 15% PAGE to give 124 bp DNA fragment, Fra-B-7. The synthesis protocol for Fra-B-7 is shown in Figure 16.

B.2 Construction of Plasmid pCdγ

The 124 bp DNA fragment (Fra-B-7) was ligated into the 4510 bp HpaI-EcoRI restriction fragment of plasmid pγtrp. The ligation mixture was used to transform E. coli HB101 and $Amp^R$ transformants were isolated. Plasmid pCdγ was isolated from the $Amp^R$ transformants and the presence of ClaI-BamHI (543 bp), ClaI-HindIII (273 bp), and AatII-ClaI (180 bp) restriction fragments were confirmed by restriction analysis. The schematic construction protocol of said plasmid is represented in Figure 5.

Preparation 5

Construction and Cloning of Plasmid pCdγtrpSd

Fra-B-8 was isolated from plasmid pCdγ as a 542 bp ClaI-BamHI restriction fragment and ligated into the 4223 bp ClaI-BamHI restriction fragment of plasmid p322trpS (Preparation 3.C). The resultant ligation mixture was used to transform E. coli HB101 and $Amp^R$ transformants were selected. Plasmid pCdγtrpSd was isolated from the transformants and the presence of HpaI-BamHI (107 and 575 bp), ClaI-BamHI (543 bp), PstI-EcoRI (1057 bp), EcoRI-BamHI (450 bp), HindIII-BamHI (270 bp) and ClaI-HindIII (273 bp)

restriction fragments were confirmed by restriction analysis. The schematic construction protocol of said plasmid pCdγtrpSd is presented in Figure 6.

Preparation 6

Construction and Cloning of α-hANP-encoding DNA with linker DNA (Fra-B-9)

A. Preparation of α-hANP-encoding DNA with linker DNA (Fra-B-9)

The ligation and purification procedures for the preparation of Fra-B-9 were conducted in substantial accordance with the method of Preparation 3, B using 300 units of T4 DNA ligase.

For the preparation of Fra-B-9, the following 18 oligonucleotides were synthesized. Oligonucleotides AH1-AH18 were used as starting materials. The synthesis protocol for 134 bp DNA fragment (Fra-B-9) is shown in Figure 17.

Fra-B-9 Oligonucleotides for α-hANP gene with linker DNA

(1) HOApGpCpTpTpGpApApGpTpTpGpApGpCpApTpGOH (AH1)

(2) HOApApTpTpCpApTpGpCpTpCpApApCpTpTpCpAOH (AT2)

(3) HOApApTpTpCpGpGpTpApTpGpGpGpCOH (AH3)

(4) HOTpTpCpApCpCpGpCpCpCpApTpApCpCpGOH (AH4)

(5) HOGpGpTpGpApApGpCpTpApApApTpCpTOH (AH4)

(6) HOCpGpCpApGpApGpApTpTpTpApGpCOH (AH6)

(7) HOCpTpGpCpGpTpApGpApTpCpCpTpCpTOH (AH7)

(8) HOApApGpCpApApGpApGpGpApTpCpTpAOH (AH8)

(9) HOTpGpCpTpTpTpGpGpTpGpGpCpCpGpTOH (AH9)

(10) HOTpCpCpApTpApCpGpGpCpCpApCpCpAOH (AH10)

(11) HOApTpGpGpApCpCpGpCpApTpCpGpGTOH (AH11)

(12) HOpTpGpApGpCpApCpCpGpApTpGpCpGpGOH (AH12)

(13) HOGpCpTpCpApGpTpCpCpGpGpTpCpTpGOH (AH13)

(14) HOCpApGpCpCpCpApGpApCpCpGpGpApCOH (AH 14)

(15) HOGpGpCpTpGpTpApApCpTpCpTpTpTpCOH (AH15)

(16) HOTpApApCpGpGpApApApGpApGpTpTpAOH (AH16)

(17) HOCpGpTpTpApCpTpGpApTpApGOH (AH17)

(18) HOGpApTpCpCpTpApTpCpApGOH (AH18)

B. Construction and Cloning of Plasmid pCLaHtrpSd

The 134 bp DNA fragment (prepared in above A.) was ligated into the 4743 bp HindIII-BamHI restriction fragment of plasmid pCdγtrpSd (prepared in Preparation 5). The ligation mixture was used to transform E. coli HB 101 and the $Amp^R$ transformant (E. coli HI) was isolated and used for the preparation of plasmid pCLaHtrpSd which encodes Cd-LH-α-hANP (fused peptide of Cd-LH and α-hANP). The resultant plasmid was subjected to the restriction analysis to confirm the presence of AatII-ClaI (287 bp), ClaI-BamHI (407 bp), ClaI-EcoRI (93 and 198 bp), EcoRI-BamHI (116 and 198 bp), HindIII-BamHI (134 bp) and HpaI-BamHI (107 and 439 bp) restriction fragments. The construction protocol for the plasmid pCLaHtrpSd is presented in Fig. 6.

Preparation 7

Construction of LH Expression Vector pLHtrp

LH gene (Fra-B-4, prepared in Preparation 1,A.2) was ligated into 4.1 kbp EcoRI-BamHI restriction fragment of plasmid pTrpEB7 (prepared in Preparation 2). The ligation mixture was used to transform E. coli HB 101 and $Amp^S$ and $Tet^R$ transformants were obtained. Plasmid pLHtrp was isolated from the transformants and the presence of the 236 bp DNA fragment (Fra-B-6) was confirmed by EcoRI-BamHI digestion and 7.5% agarose gel electrophresis. The construction protocol is presented in Fig. 7.

Preparation 8

IGF-I Expression Vector pLHSdMmtrp

A. Preparation of Gene Encoding IGF-I (Fra-B-10)

A.1. Ligation of Synthetic Oligonucleotides

For the preparation of Fra-B-10, the following 30 oligonucleotides were synthesized.

Oligonucleotides for IGF-I gene, Fra-B-10

(1) HOApApTpTpCpApTpGpGpGpTOH (A1)

(2) HOTpTpTpCpApGpGpApCpCpCpApTpGOH (A2)

(3) HOCpCpTpGpApApApCpTpCpTpGpTpGOH (B1)

(4) HOCpApGpCpGpCpCpGpCpApCpApGpApGOH (B2)

(5) HOCpGpGpCpGpCpTpGpApApCpTpGpGpTOH (C1)

(6) HOApGpApGpCpGpTpCpApApCpCpApGpTpTOH (C2)

(7) HOTpGpApCpGpCpTpCpTpGpCpApApTpTpTOH (D1)

(8) HOCpCpApCpApTpApCpApApApTpTpGpCOH (D2)

(9) HOGpTpApTpGpTpGpGpTpGpApTpCpGpTOH (E1)

(10) HOTpApGpApApApCpCpApCpGpApTpCpAOH (E2)

(11) HOGpGpTpTpTpCpTpApCpTpTpCpApApCOH (F1)

(12) HOGpGpTpCpGpGpTpTpTpGpTpTpGpApApGOH (F2)

(13) HOApApApCpCpGpApCpCpGpGpCpTpApTpGOH (G1)

(14) HOGpCpTpGpGpApGpCpCpApTpApGpCpCOH (G2)

(15) HOGpCpTpCpCpApGpCpTpCpTpCpGpTpCOH (H1)

(16) HOCpGpGpTpGpCpGpCpGpApCpGpApGpAOH (H2)

(17) HOGpCpGpCpApCpCpGpCpApGpApCpTpGOH (I1)

(18) HOCpTpApCpGpApTpApCpCpApGpTpCpTpGOH (I2)

(19) HOGpTpApTpCpGpTpApGpApCpGpApApTpGOH (J1)

(20) HOGpApApApApCpApGpCpApTpTpCpGpTOH (J2)

(21) HOCpTpGpTpTpTpTpCpGpTpTpCpTpTpGOH (K1)

(22) HOGpGpApGpApTpCpGpCpApApGpApApCOH (K2)

(23) HOCpGpApTpCpTpCpCpGpCpCpGpTpCpTOH (L1)

(24) HOTpApCpApTpTpTpCpCpApGpApCpGpGpCOH (L2)

(25) HOGpGpApApApTpGpTpApCpTpGpTpGpCpTOH (M1)

(26) HOTpTpCpApGpTpGpGpApGpCpApCpApGOH (M2)

(27) HOCpCpApCpTpGpApApGpCpCpApGpCpAOH (N1)

(28) HOGpCpGpGpApTpTpTpTpGpCpTpGpGpCOH (N2)

(29) HOApApApTpCpCpGpCpGpTpGpApTpApGOH (O1)

(30) HOGpApTpCpCpTpApTpCpApCOH (O2)

Each oligonucleotide (A1-O2, Fig. 18) (each 0.4nM) was phosphorylated with T4 polynucleotide kinase in 100μl of ligation buffer (74mM Tris-HCl, pH 7.6, 10mM DTT, 1.6mM mercaptoethanol, 10mM $MgCl_2$ and 0.5mM ATP) at 37°C for 20 minutes. When the reaction was completed, the reaction mixture was incubated at 100°C for 5 minutes to inactivate the enzyme. In accordance with the process in Fig. 18, the IGF-I-encoding DNA was prepared for cloning. The ligation was conducted in a reaction mixture containing 7 units of T4 DNA ligase and 0.5μl of 100mM ATP at 4°C for 23 hours. Each ligation product was separated by 2-16% gradient PAGE in Tris-EDTA buffer and visualized by ethidium bromide.

A.2. Cloning of 224 bp DNA Fragment Encoding IGF-I

Fra-B-10 was ligated into the 3985 bp BamHI-EcoRI restriction fragment of plasmid pBR322 at 12°C for 18 hours. The resultant ligation mixture was used to transform E. coli HB101 in substantial accordance with the procedure of Kushner's method (supra) and the $Amp^R$ transformants were selected on agar plate containing 25μg/ml of tetracycline. The presence of desired 224 bp DNA fragment was confirmed by EcoRI and BamHI digestion of the plasmid which has been isolated from the $Amp^R$ and $Tet^S$ clones and the comparison of the restriction fragments with appropriate size markers.

Desired plasmid, designated pSdM1, was characterized by the existence of Met-IGF-I encoding gene by restriction analysis.

B. Construction of IGF-I Expression Vector pLHSdMtrp

For the preparation of Fra-B-11, the following oligonucleotides were synthesized.

Fra-B-11 IGF-I gene with linker DNA containing internal SD sequence

(1) HOApGpCpTpTpGpApApGpTpApApApApCpApTpGOH (m1)

(2) HOApApTpTpCpApTpGpTpTpTpTpApCpTpTpCpAOH (m2)

Oligonucleotide m2 was phosphorylated in accordance with the method of Preparation 8.A.1. Phosphorylated oligonucleotide m2 and unphosphorylated oligonucleotide m1 were ligated to the 224 bp EcoRI-BamHI restriction fragment of plasmid pSdM1 (above A.2) with T4 polynucleotide kinase in the ligation buffer containing 100mM ATP at 4°C for 20 hours. After digestion with BamHI, the ligation mixture was purified on PAGE to isolate the IGF-I-encoding DNA with linker DNA (242 bp, Fra-B-11). Fra-G-11 was ligated to the HindIII-BamHI restriction fragment of pLHtrp and the ligation mixture was used to transform E. coli HB 101. The host cell containing the transforming plasmid was designated E. coli F-6. From this transformants was isolated pLHSdMmtrp, and the plasmid was subjected to the restriction analysis. On the 7.5% PAGE, the presence of EcoRI-BamHI (198 and 224 bp), HindIII-BamHI (242 bp) and HpaI-BamHI (456

bp) fragments were confirmed. The construction protocol of said plasmid is presented in Figure 7. The synthesis protocol of Fra-B-10 is shown in Figure 18.

Preparation 9

Construction and Cloning of Plasmid pBR322trpSs Encoding Trp Promoter III

Trp promoter III-encoding DNA (prepared in Preparation 3.B.) was ligated into the 4340 bp EcoRI-ClaI restriction fragment of plasmid pBR322 in the presence of 1mM ATP. The ligation mixture was used to transform E. coli HB 101, and plasmid pBR322trpSs was isolated from the transformants. The presence of HpaI (4445 bp) and ClaI-PstI (834 bp) restriction fragments was confirmed. The schematic construction protocol of said plasmid pBR322trpSs is presented in Figure 10.

Preparation 10

Construction of Plasmid pCLaHtrp-2

The 406 bp ClaI-BamHI restriction fragment (Fra-C-2) of plasmid pCLaHtrpSd (prepared in Preparation 6.B) was ligated into the 4093 bp ClaI-BamHI restriction fragment of pBR322trpSs (prepared in Preparation 9), and the ligation mixture was used to transform E. coli HB 101. The desired plasmid pCLaHtrp-2 was isolated from the transformants and characterized by restriction analysis: ClaI-PstI (834 bp) and ClaI-BamHI (406 bp). The schematic construction protocol of said plasmid pCLaHtrp-2 is presented in Figure 10.

Preparation 11

Construction of α-hANP Expression Vector pCLaHtrp3t bearing Fd Phage Terminator

A. Synthesis of Fd Phage Terminator and Cloning thereof

Fd phage terminator (Fra-C-1) was synthesized substantially in accordance with the method of Preparation 3.B using following 6 oligonucleotides:

Fra-C-1 Synthetic fd phage terminator gene

(1) HOGpApTpCpCpTpCpGpApGpApTpCpApAOH (T1)

(2) HOGpCpCpTpTpTpApApTpTpGpApTpCpTpCpGpApGOH (T2)

(3) HOTpTpApApApGpGpCpTpCpCpTpTpTpTpGpGpAOH (T3)

(4) HOApApApApApGpGpCpTpCpCpApApApApGpGpAOH (T4)

(5) HOGpCpCpTpTpTpTpTpTpTpTpTpTpGOH (T5)

(6) HOTpCpGpApCpApApApApAOH (T6)

Oligonucleotides T2, T3, T4 and T5, each 0.4nM, were mixed and phosphorylated with T4 polynucleotide kinase in the presence of 1mM ATP. The reaction mixture was incubated at 65 °C for 10 minutes to inactivate the enzyme. To the mixture were added oligonucleotides T1 and T6 (0.8nM each) and incubated at 15 °C for 30 min in the presence of T4 DNA ligase. The ligation mixture was fractionated on 2-16% gradient polyacrylamide gel, and the desired 47 bp restriction fragment was recovered. The fragment was then ligated into the 4088 bp BamHI-SalI restriction fragment of plasmid pBR322 to form plasmid pter21. Plasmid pter21 was subjected to the restriction analysis to confirm the presence of BamHI-SalI (47 bp) and AvaI (817 bp) restriction fragments.

B. Construction and Cloning of Plasmid pCLaHtrp3t

The 1241 bp PstI-BamHI restriction fragment of plasmid pCLaHtrp-2 (prepared in Preparation 10) was ligated into the 3005 by PstI-BamHI restriction fragment of plasmid pter21. The resultant ligation mixture was used to transform E. coli HB 101 to give E. coli H2 resistant to ampicillin, from which was isolated plasmid pCLaHtrp3t. The plasmid was then subjected to the restriction analysis to confirm the presence of ClaI-EcoRI (939 and 198 bp), HindIII-BamHI (134 bp), and PstI-ClaI-XhaI (834 and 411 bp) restriction fragments. The schematic construction protocol of plasmid pCLaHtrp3t is presented in Figure 11.

Example 1

Construction of Met-IGF-I Expression Vector pCE-SMtrp

A. Preparation of Fra-A-7 for the Construction of Two-cistronic Vector

The following oligonucleotides were synthesized for the construction of the DNA fragment.

Fra-A-1 and Fra-A-3 Linker DNA containing internal SD sequence

(1) HOGpTpTpGpCpCpApGpTpApCpCpGpCpGpAOH (S)

(2) HOTpTpCpApGpGpTpCpGpCpGpGpTpApCOH (T)

(3) HOCpCpTpGpApApGpCpTpTpGpApGpGpAOH (CT1)

(4) HOCpGpApTpTpCpTpCpCpTpCpApApGpCOH (CT2)

(5) HOGpApApTpCpGpApTpApApTpGpTpCpTOH (CT3)

(6) HOCpGpCpApGpApGpApCpApTpTpApTOH (CT4)

(7) HOCpGpApTpApApTpGpGOH (CT5)

(8) HOGpApCpCpCpApTpTpApTOH (CT6)

Oligonucleotides S, T, $CT_1$, $CT_2$, $CT_3$ and $CT_4$ (each 0.4nM) were phosphorylated with T4 polynucleotide kinase (5U, Takara Syuzou, Inc.) in 130µl of ligation buffer (50mM Tris-HCl, pH 7.6, 20mM DTT, 1mM spermidine, 50µg of BSA, 10mM $MgCl_2$ and 1mM ATP) at 37°C for 1 hour. To the mixture were added 3µl of 20mM ATP and 875 units of T4 DNA ligase (Takara Syuzou, Inc.) and the mixture was incubated at 15°C for 30 minutes, and then at 65°C for 20 minutes, to inactivate the enzyme. The ligation mixture containing 46 bp DNA fragment (Fra-A-1) was incubated with 14µl of 500mM NaCl and 10 units of ClaI at 37°C for 2 hours and the resultant mixture was purified on 2-16% gradient PAGE to obtain 300ng of 35 bp DNA fragment (Fra-A-2).

A.2. Preparation of 44 bp DNA Fragment (Fra-A-3)

Fra-A-2 (300ng), $CT_5$ (0.4nM), and $CT_6$ (0.4nM) were incubated in the presence of 350 units of T4 DNA ligase in 20 μl of ligation buffer at 15° C for 30 minutes. The ligation mixture was purified by 2.25% agarose gel electrophoresis (2.25% AGE) to give 100ng of 44 bp DNA Fragment (Fra-A-3).

A.3. Preparation of Fra-A-5

Plasmid pLHSdMmtrp (10μg, prepared in Preparation 8, 4.5 kbp) was digested with HindIII and BamHI to yield 300ng of the 242 bp DNA fragment encoding IGF-I (Fra-A-4), which was then cleaved with AvaII to give 100ng of 215 bp DNA fragment (Fra-A-5).

A.4 Preparation of Fra-A-7

Fra-A-3 (100ng), Fra-A-5 (100ng) and 350 units of T4 DNA ligase were incubated overnight in 12 μl of ligation buffer at 4° C. After heating at 65° C for 10 minutes, the reaction mixture containing Fra-A-6 was treated with 5 units of each of restriction enzymes BamHI and HindIII. The reaction mixture was purified on 2.25% PAGE to recover 30ng of 238 bp DNA fragment (Fra-A-7).

B. Construction of plasmid pCE-SMtrp

About 5μg of HindIII-BamHI restriction fragment was isolated from 10μg of plasmid pCLaHtrpSd (prepared in Preparation 6), and 200ng of the fragment was ligated to Fra-A-7 in the presence of 350 units of T4 DNA ligase in 20 μl of ligation buffer at 15° C for 30 minutes. The resultant ligation mixture was used to transform E. coli DH-1. Plasmid pCE-SMtrp was isolated from $Amp^r$ transformants and confirmed the presence of ClaI-EcoRI (93 and 193 bp), EcoRI-HindIII (180 bp), HindIII-BamHI (238 bp), HpaI-BamHI (107 and 544 bp) restriction fragments.The construction protocol is presented in Figure 9. The plasmid pCE-SMtrp was transformed into E. coli HB 101 to form the transformant E. coli HB 101/pCE-SMtrp.

Example 2

Construction of Plasmid pCE-SM3t

The 238 bp HindIII-BamHI restriction fragment of plasmid pCE-SMtrp (Fra-A-7) was ligated into the 4137 bp HindIII-BamHI restriction fragment of plasmid pClaHtrp3t (prepared in Preparation 11) and the ligation mixture was used to transform E. coli HB 101. Recombinant plasmid was isolated from the clone of transformant and confirmed the existence of 286 bp ClaI restriction fragment by restriction analysis. The construction protocol of plasmid pCE-SM3t is presented in Figure 12.

Example 3

Isolation of Met-IGF-I from E. coli HB 101/pCE-SMtrp

A. Expression of IGF-I-encoding Gene

A single colony of transformant E. coli HB 101/pCE-SMtrp was grown in L medium (5ml of LA broth) containing 50 μg/ml ampicillin and incubated at 37° C. After 8 hours cultivation, the culture was added to 100ml of LA medium and incubated overnight at 37° C. The overnight culture (20ml) was added to 400ml of M9CA medium (0.5% casamino acid, 0.2% glucose, 50μg/ml thiamine HCl, and 25μg/ml ampicillin) and incubated at 37° C until the optical density (600 nanometers, A 600) reached 0.5 absorbance unit. To the medium was added 2 ml of IAA (2mg indoleacrylic acid / ml ethanol), and the culture was incubated for further 3 hours (final A 600 = 1.40), followed by centrifugation at 6,000rpm for 5 minutes to collect the cells.

B. Isolation and Purification of Protein

Collected wet cells were resuspended in 8ml of 10mM PBS-EDTA buffer (containing 8.0g NaCl, 0.2g KCl, 2.9g $Na_2HPO_4 \cdot 12H_2O$, 0.2g $KH_2PO_4$, 3.73g EDTA in 1L and adjusted pH 8.0 with NaOH) and disrupted by ultrasonification, and the resultant suspension was centrifuged at 15,000rpm for 20 minutes at

4°C. The supernatant was discarded and the precipitate was resuspended in Gn•HCl buffer (6M guanidine HCl, 10mM PBS-EDTA, 2mM β-mercaptoethanol), and the suspension was ultrasonificated at 0°C. After centrifugation of the suspension at 15,000 rpm at 4°C for 20 min, the supernatant was dialyzed 3 times against each 1000ml of 1M AcOH. Thirty ml of acetone was then added to the dialysed solution and the mixture was allowed to stand for 10 min at -78°C followed by centrifugation at 15,000 rpm for 20 minutes at 4°C. The resultant precipitate was dried under reduced pressure to give crude Met-IGF-I, which was then dissolved in Gn•HCl buffer containing 10% β-mercaptoethanol, and the solution was subjected to the reverse HPLC for further purification (column; Beckman RPSC: flow rate; 1ml/min: eluent; 0.08% trifluoroacetic acid (TFA) - 10%

$$CH_3CN \xrightarrow{50min} 0.08\%$$

TFA - 60% $CH_3CN$: wave length; 214nm). Yield = 1.6mg.

C. Identification of Expressed Protein

The purified protein was identified as Met-IGF-I by N-terminal analysis and peptide mapping of the protein which had been treated with chymotrypsin.

**Claims**

**Claims for the following Contracting States : AT, BE, CH, DE, FR, GB, IT, LI, LU, NL, SE**

1. A recombinant DNA expression vector for the production in Escherichia coli of Met-IGF-I, said vector comprises:
   a) a promoter-encoding gene;
   b) the first cistron comprising a gene encoding a protective peptide with a molecular weight of about 500-50,000, said gene containing a translational initiation signal immediately adjacent to the codon encoding the N-terminus of said peptide and a translational termination signal positioned downstream from the codon encoding the C-terminus of said peptide;
   c) the second cistron comprising a gene encoding IGF-I, said gene containing a translational initiation signal immediately adjacent to the codon encoding the N-terminus of IGF-I and a translational termination signal downstream from the codon encoding the C-terminus of IGF-I; subject to the limitation that said vector contains two SD sequences each located upstream from the codon for the N-terminus of each peptide, and that said vector is selectable and autonomously replicable in E. coli.

2. The vector of Claim 1 wherein the protective peptide is an acidic peptide.

3. The vector of Claim 1 wherein the protective peptide is Cd-LH.

4. The vector of any of Claims 1 to 3 in which the translational termination signal contained in the first cistron and the translational initiation signal contained in the second cistron are overlapped, or immediately adjacent each other, or separated by one or two intervening nucleotides.

5. The vector of any of Claims 1 to 4 in which the promoter is a tryptophan operon promoter or its derivative.

6. The vector of any of Claim 5 in which the overlapped nucleotide sequence of translational termination signal in the first cistron and the translational initiation signal in the second cistron is TAATG, wherein A is deoxyadenyl, G is deoxyguanyl, and T is thymidyl.

7. The vector of any of Claims 1 to 6 in which the translational termination signal of the second cistron is derived from plasmid pBR322 or fd phage.

8. The vector of any of Claims 1 to 7 which is plasmid.

**9.** The vector of any of Claims 1 to 8 which is plasmid pCE-SMtrp shown by the Fig. 2(1) or pCE-SM3t shown by the Fig. 2(2).

**10.** A transformed host cell bearing any of the vector of Claims 1 to 9.

**11.** The transformed host cell of Claim 10 which is Escherichia coli.

**12.** The transformed host cell of Claim 11 which is E. coli HB 101.

**13.** A method for preparing Met-IGF-I, which comprises:
a) transforming a host cell with a vector as claimed in any of Claims 1 to 9;
b) growing said transformed host cell under conditions suitable for expression;
c) lysing the transformed cell; and
d) isolating Met-IGF-I from the lysate.

**Claims for the following Contracting States : ES, GR**

**1.** A method for preparing Met-IGF-I, which comprises:
a) transforming a host cell with a recombinant DNA expression vector for the production in Escherichia coli of Met-IGF-I, said vector comprises:
i) a promoter-encoding gene;
ii) the first cistron comprising a gene encoding a protective peptide with a molecular weight of about 500-50,000, said gene containing a translational initiation signal immediately adjacent to the codon encoding the N-terminus of said peptide and a translational termination signal positioned downstream from the codon encoding the C-terminus of said peptide;
iii) the second cistron comprising a gene encoding IGF-I, said gene containing a translational initiation signal immediately adjacent to the codon encoding the N-terminus of IGF-I and a translational termination signal downstream from the codon encoding the C-terminus of IGF-I;
subject to the limitation that said vector contains two SD sequences each located upstream from the codon for the N-terminus of each peptide, and that said vector is selectable and autonomously replicable in E. coli;
b) growing said transformed host cell under conditions suitable for expression;
c) lysing the transformed cell; and
d) isolating Met-IGF-I from the lysate.

**2.** The method of Claim 1 wherein the protective peptide is an acidic peptide.

**3.** The method of Claim 1 wherein the protective peptide is Cd-LH.

**4.** The method of any of Claims 1 to 3 in which the translational termination signal contained in the first cistron and the translational initiation signal contained in the second cistron are overlapped, or immediately adjacent each other, or separated by one or two intervening nucleotides.

**5.** The method of any of Claims 1 to 4 in which the promoter is a tryptophan operon promoter or its derivative.

**6.** The method of any of Claim 5 in which the overlapped nucleotide sequence of translational termination signal in the first cistron and the translational initiation signal in the second cistron is TAATG, wherein A is deoxyadenyl, G is deoxyguanyl, and T is thymidyl.

**7.** The method of any of Claims 1 to 6 in which the translational termination signal of the second cistron is derived from plasmid pBR322 or fd phage.

**8.** The method of any of Claims 1 to 7 which is plasmid.

**9.** The method of any of Claims 1 to 8 which is plasmid pCE-SMtrp shown by the Fig. 2(1) or pCE-SM3t shown by the Fig. 2(2).

**Patentansprüche**

**Patentansprüche für folgende Vertragsstaaten : AT, BE, CH, DE, FR, GB, IT, LI, LU, NL, SE**

1. Rekombinanter DNA-Expressionsvektor zur Produktion von Met-IGF-I in Escherichia coli, wobei der Vektor umfaßt:
   a) ein Promoter-codierendes Gen;
   b) das erste Cistron, umfassend ein für ein Schutzpeptid mit einem Molekulargewicht von etwa 500 - 50000 codierendes Gen, wobei das Gen ein Translations-Initiationssignal unmittelbar benachbart zu dem Codon enthält, der für den N-Terminus des Peptides codiert, und ein Translations-Terminationssignal, das stromabwärts von dem Codon angeordnet ist, der für den C-Terminus des Peptides codiert;
   c) das zweite Cistron, umfassend ein für IGF-I codierendes Gen, wobei das Gen ein Translations-Initiationssignal unmittelbar benachbart zu dem Codon enthält, der für den N-Terminus von IGF-I codiert, und ein Translations-Terminationssignal, das stromabwärts von dem Codon angeordnet ist, der für den C-Terminus von IGF-I codiert;
   wobei der Vektor als Gegenstand der Beschränkung zwei SD-Sequenzen enthält, die jeweils stromaufwärts von dem Codon für den N-Terminus eines jeden Peptides angeordnet sind, und wobei der Vektor selektierbar und autononom replizierbar in E. coli ist.

2. Vektor nach Anspruch 1, worin das Schutzpeptid ein saures Peptid ist.

3. Vektor nach Anspruch 1, worin das Schutzpeptid Cd-LH ist.

4. Vektor nach einem der Ansprüche 1 bis 3, worin das in dem ersten Cistron enthaltene Translations-Terminationssignal sich mit dem in dem zweiten Cistron enthaltenen Translations-Terminationssignal überlappt, oder beide unmittelbar benachbart sind oder voneinander getrennt sind durch ein oder zwei intervenierende Nucleotide.

5. Vektor nach einem der Ansprüche 1 bis 4, worin der Promotor ein Tryptophan-Operon Promoter oder dessen Derivat ist.

6. Vektor nach einem der Ansprüche 1 bis 5, worin die überlappende Nucleotidsequenz des Translations-Terminationssignals in dem ersten Cistron und des Translations-Initiationssignals in dem zweiten Cistron TAATG ist, worin A Desoxyadenyl ist, G ist Desoxyguanyl und T ist Thymidyl.

7. Vektor nach einem der Ansprüche 1 bis 6, worin das Translations-Terminationssignaldes zweiten Cistrons abgeleitet ist aus dem Plasmid pBR322 oder fd-Phage.

8. Vektor nach einem der Ansprüche 1 bis 7, der ein Plasmid ist.

9. Vektor nach einem der Ansprüche 1 bis 8, der das Plasmid pCE-SMtrp [gemäß Fig. 2(1)] oder pCE-SM3t [gemäß Fig. 2(2)] ist.

10. Transformierte Wirtszelle, die einen der Vektoren der Ansprüche 1 bis 9 trägt.

11. Transformierte Wirtszelle nach Anspruch 10, die Escherichia coli ist.

12. Transformierte Wirtszelle nach Anspruch 11, die E. coli HB 101 ist.

13. Verfahren zur Herstellung von Met-IGF-I, das umfaßt
   a) Transformieren einer Wirtszelle mit einem Vektor, wie er in einem der Ansprüche 1 bis 9 beansprucht wurde;
   b) Wachstum der transformierten Wirtszelle unter für die Expression geeigneten Bedingungen;
   c) Lysieren der transformierten Zelle; und
   d) Isolieren von Met-IGF-I aus dem Lysat.

**Patentansprüche für folgende Vertragsstaaten : ES, GR**

1. Verfahren zur Herstellung von Met-IGF-I, das umfaßt
   a) Transformieren einer Wirtszelle mit einem rekombinanten DNA-Expressionsvektor für die Produktion von Met-IGF-I in Escherichia coli, wobei der Vektor umfaßt :
      i) ein Promoter-codierendes Gen;
      ii) das erste Cistron, umfassend ein für ein Schutzpeptid mit einem Molekulargewicht von etwa 500 - 50000 codierendes Gen, wobei das Gen ein Translations-Initiationssignal unmittelbar benachbart zu dem Codon enthält, der für den N-Terminus des Peptides codiert, und ein Translations-Terminationssignal, das stromabwärts von dem Codon angeordnet ist, der für den C-Terminus des Peptides codiert;
      iii) das zweite Cistron, umfassend ein für IGF-I codierendes Gen, wobei das Gen ein Translations-Initiationssignal unmittelbar benachbart zu dem Codon enthält, der für den N-Terminus von IGF-I codiert, und ein Translations-Terminationssignal, das stromabwärts von dem Codon angeordnet ist, der für den C-Terminus von IGF-I codiert;
      wobei der Vektor als Gegenstand der Beschränkung zwei SD-Sequenzen enthält, die jeweils stromaufwärts von dem Codon für den N-Terminus eines jeden Peptides angeordnet sind, und wobei der Vektor selektierbar und autononom replizierbar in E. coli ist;
   b) Wachstum der transformierten Wirtszelle unter für die Expression geeigneten Bedingungen;
   c) Lysieren der transformierten Zelle; und
   d) Isolieren von Met-IGF-I aus dem Lysat.

2. Verfahren nach Anspruch 1, worin das Schutzpeptid ein saures Peptid ist.

3. Verfahren nach Anspruch 1, worin das Schutzpeptid Cd-LH ist.

4. Verfahren nach einem der Ansprüche 1 bis 3, worin das in dem ersten Cistron enthaltene Translations-Terminationssignal sich mit dem in dem zweiten Cistron enthaltenen Translations-Terminationssignal überlappt, oder beide unmittelbar benachbart sind oder voneinander getrennt sind durch ein oder zwei intervenierende Nucleotide.

5. Verfahren nach einem der Ansprüche 1 bis 4, worin der Promotorü ein Tryptophan-Operon Promoter oder dessen Derivat ist.

6. Verfahren nach einem der Ansprüche 1 bis 5, worin die überlappende Nucleotidsequenz des Translations-Terminationssignals in dem ersten Cistron und des Translations-Initiationssignals in dem zweiten Cistron TAATG ist, worin A Desoxyadenyl ist, G ist Desoxyguanyl und T ist Thymidyl.

7. Verfahren nach einem der Ansprüche 1 bis 6, worin das Translations-Terminationssignal des zweiten Cistrons abgeleitet ist aus dem Plasmid pBR322 oder fd-Phage.

8. Verfahren nach einem der Ansprüche 1 bis 7, der ein Plasmid ist.

9. Verfahren nach einem der Ansprüche 1 bis 8, der das Plasmid pCE-SMtrp [gemäß Fig. 2(1)] oder pCE-SM3t [gemäß Fig. 2(2)] ist.

## Revendications
## Revendications pour les Etats contractants suivants : AT, BE, CH, DE, FR, GB, IT, LI, LU, NL, SE

1. Vecteur d'expression de l'ADN recombiné pour la production dans Escherichia coli de Met-IGF-I, ce vecteur comprenant :
   a) un gène codant pour un promoteur;
   b) le premier cistron comprenant un gène codant pour un peptide protecteur avec une masse moléculaire d'environ 500 à 50 000, ce gène contenant un signal d'initiation de la traduction immédiatement adjacent au codon codant pour l'extrémité N-terminale de ce peptide et un signal de terminaison de la traduction disposé en aval du codon codant pour l'extrémité C-terminale de ce peptide;
   c) le second cistron comprenant un gène codant pour IGF-I, ce gène contenant un signal d'initiation de la traduction immédiatement adjacent au codon codant pour l'extrémité N-terminale de l'IGF-I, et un signal de terminaison de la traduction en aval du codon codant pour l'extrémité C-terminale de

l'IGF-I; sous-réserve que ce vecteur contienne deux séquences SD situées chacune en amont du codon pour l'extrémité N-terminale de chaque peptide, et que ce vecteur soit sélectionnable et réplicable d'une manière autonome dans E. coli.

**2.** Vecteur selon la revendication 1, dans lequel le peptide protecteur est un peptide acide.

**3.** Vecteur selon la revendication 1, dans lequel le peptide protecteur est Cd-LH.

**4.** Vecteur selon l'une quelconque des revendications 1 à 3, dans lequel le signal de terminaison de la traduction contenu dans le premier cistron et le signal d'initiation de la traduction contenu dans le second cistron se chevauchent ou sont immédiatement adjacents l'un à l'autre, ou séparés par un ou deux nucléotides intercalaires.

**5.** Vecteur selon l'une quelconque des revendications 1 à 4, dans lequel le promoteur est un promoteur de l'opéron du tryptophane ou son dérivé.

**6.** Vecteur selon la revendication 5, dans lequel la séquence de nucléotide chevauchée du signal de terminaison de la traduction dans le premier cistron et le signal d'initiation de la traduction dans le second cistron est TAATG, où A est un groupe désoxyadényle, G est un groupe désoxyguanyle et T est un groupe thymidyle.

**7.** Vecteur selon l'une quelconque des revendications 1 à 6, dans lequel le signal de terminaison de la traduction du second cistron est obtenu à partir du plasmide pBR322 ou du phage fd.

**8.** Vecteur selon l'une quelconque des revendications 1 à 7, qui est un plasmide.

**9.** Vecteur selon l'une quelconque des revendications 1 à 8, qui est un plasmide pCE-SMtrp représenté par la figure 2(1) ou pCE-SM3t représenté par la figure 2(2).

**10.** Cellule hôte transformée portant l'un quelconque des vecteurs selon la revendication 1 à 9.

**11.** Cellule hôte transformée selon la revendication 10, qui est Escherichia coli.

**12.** Cellule hôte transformée selon la revendication 11, qui est E. coli HB 101.

**13.** Procédé de préparation de Met-IGF-I, qui comprend :
a) la transformation d'une cellule hôte avec un vecteur selon l'une quelconque des revendications 1 à 9;
b) la culture de cette cellule hôte transformée dans des conditions convenant pour l'expression;
c) la lyse de la cellule transformée; et
d) l'isolement de Met-IGF-I du lysat .

**Revendications pour les Etats contractants suivants : ES, GR**

**1.** Procédé de préparation de Met-IGF-I, qui comprend :
a) la transformation d'une cellule hôte avec un vecteur d'expression ADN recombinant pour la production dans Escherichia coli de Met-IGF-I, ce vecteur comprenant :
i) un gène codant pour un promoteur;
ii) le premier cistron comprenant un gène codant pour un peptide protecteur ayant une masse moléculaire d'environ 500 à 50 000, ce gène contenant un signal d'initiation de la traduction immédiatement adjacent au codon codant pour l'extrémité N-terminale de ce peptide et un signal de terminaison de la traduction disposé en aval du codon codant pour l'extrémité C-terminale de ce peptide;
iii) le second cistron comprenant un gène codant pour l'IGF-I, ce gène contenant un signal d'initiation de la traduction immédiatement adjacent au codon codant pour l'extrémité N-terminale de l'IGF-I, et un signal de terminaison de la traduction en aval du codon codant pour l'extrémité C-terminale de l'IGF-I; sous-réserve que ce vecteur contienne deux séquences SD situées chacune en aval du codon pour l'extrémité N-terminale de chaque peptide et que ce vecteur soit

sélectionnable et réplicable d'une manière autonome dans E. coli.
b) la culture de cette cellule hôte transformée dans des conditions convenant pour l'expression;
c) la lyse de la cellule transformée; et
d) l'isolation de Met-IGF-I du lysat .

**2.** Procédé selon la revendication 1, dans lequel le peptide protecteur est un peptide acide.

**3.** Procédé selon la revendication 1, dans lequel le peptide protecteur est Cd-LH.

**4.** Procédé selon l'une quelconque des revendications 1 à 3, dans lequel le signal de terminaison de la traduction contenu dans le premier cistron et le signal d'initiation de la traduction contenu dans le second cistron se chevauchent ou sont immédiatement adjacents l'un à l'autre, ou séparés par un ou deux nucléotides intercalaires.

**5.** Procédé selon l'une quelconque des revendications 1 à 4, dans lequel le promoteur est un promoteur de l'opéron du tryptophane ou son dérivé.

**6.** Procédé selon la revendication 5, dans lequel la séquence de nucléotide chevauchée du signal de terminaison de la traduction dans le premier cistron et le signal d'initiation de la traduction dans le second cistron est TAATG, où A est un groupe désoxyadényle, G est un groupe désoxyguanyle et T est un groupe thymidyle.

**7.** Procédé selon l'une quelconque des revendications 1 à 6, dans lequel le signal de terminaison de la traduction du second cistron est obtenu à partir du plasmide pBR322 ou du phage fd.

**8.** Procédé selon l'une quelconque des revendications 1 à 7, qui est un plasmide.

**9.** Procédé selon l'une quelconque des revendications 1 à 8, qui est un plasmide pCE-SMtrp représenté par la figure 2(1) ou pCE-SM3t représenté par la figure 2(2).

*Fig. 1*

```
                                     1
                            EcoRI Met GlY Pro Glu Thr Leu
                          5'-AATTC-ATG-GGT-CCT-GAA-ACT-CTG-
                              3'-G-TAC-CCA-GGA-CTT-TGA-GAC-

             10
Cys Gly Ala Glu Leu Val Asp Ala Leu Gln Phe Val Cys Gly
TGC-GGC-GCT-GAA-CTG-GTT-GAC-CCT-CTG-CAA-TTT-GTA-TGT-GGT-
ACG-CCG-CGA-CTT-GAC-CAA-CTG-CGA-GAC-GTT-AAA-CAT-ACA-CCA-

 20                                      30
Asp Arg Gly Phe Tyr Phe Asn Lys Pro Thr Gly Tyr Gly Ser
GAT-CGT-GGT-TTC-TAC-TTC-AAC-AAA-CCG-ACC-GGC-TAT-GGC-TCC-
CTA-GCA-CCA-AAG-ATG-AAG-TTG-TTT-GGC-TGG-CCG-ATA-CCG-AGG-

                         40
Ser Ser Arg Arg Ala Pro Gln Thr Gly Ile Val Asp Glu Cys
AGC-TCT-CGT-CGC-GCA-CCG-CAG-ACT-GGT-ATC-GTA-GAC-GAA-TGC-
TCG-AGA-GCA-GCG-CGT-GGC-GTC-TGA-CCA-TAG-CAT-CTG-CTT-ACG-

         50                                          60
Cys Phe Arg Ser Cys Asp Leu Arg Arg Leu Glu Met Tyr Cys
TGT-TTT-CCT-TCT-TGC-GAT-CTC-CGC-CGT-CTG-GAA-ATG-TAC-TGT-
ACA-AAA-GCA-AGA-ACG-CTA-GAG-GCG-GCA-GAC-CTT-TAC-ATG-ACA-

                                 70
Ala Pro Leu Lys Pro Ala Lys Ser Ala stop stop BamHI
GCT-CCA-CTG-AAG-CCA-GCA-AAA-TCC-GCG-TGA-TAG-3'
CGA-GGT-GAC-TTC-GGT-CGT-TTT-AGG-CGC-ACT-ATC-CTAG-5'
```

Fig. 2(1)

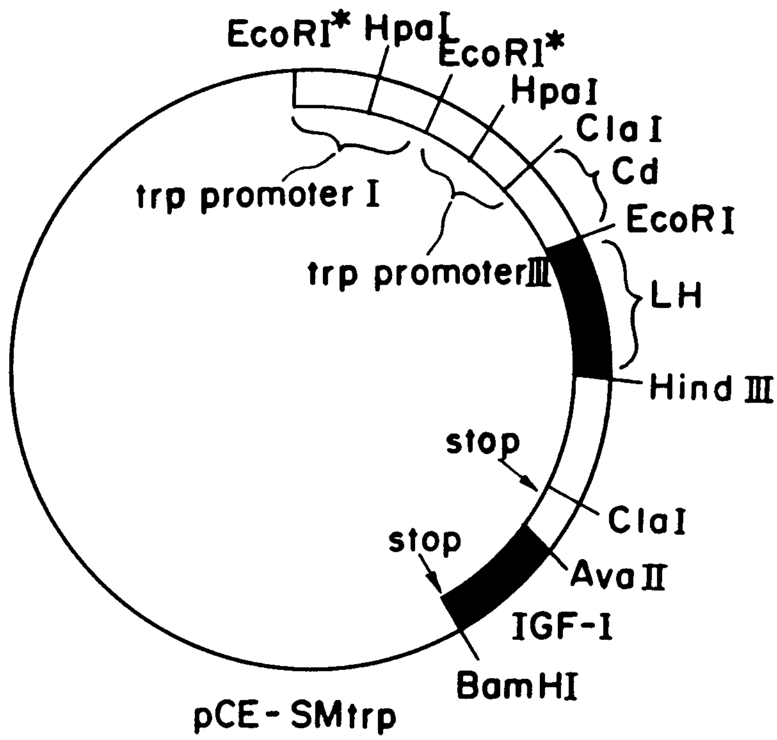


pCE-SMtrp

Fig. 2(2)

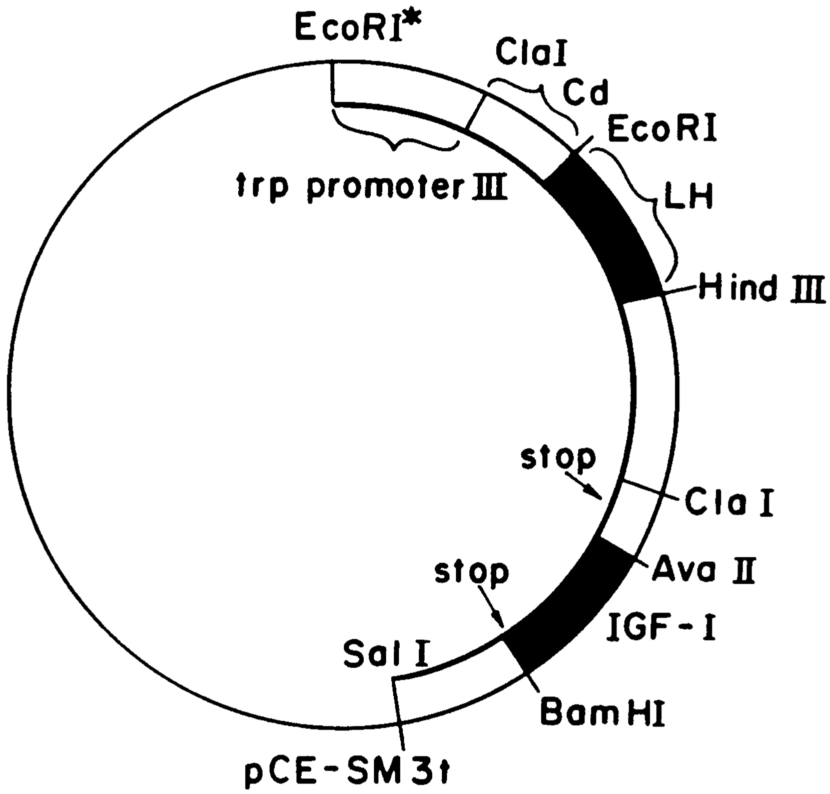


pCE-SM3t

*Fig. 3*

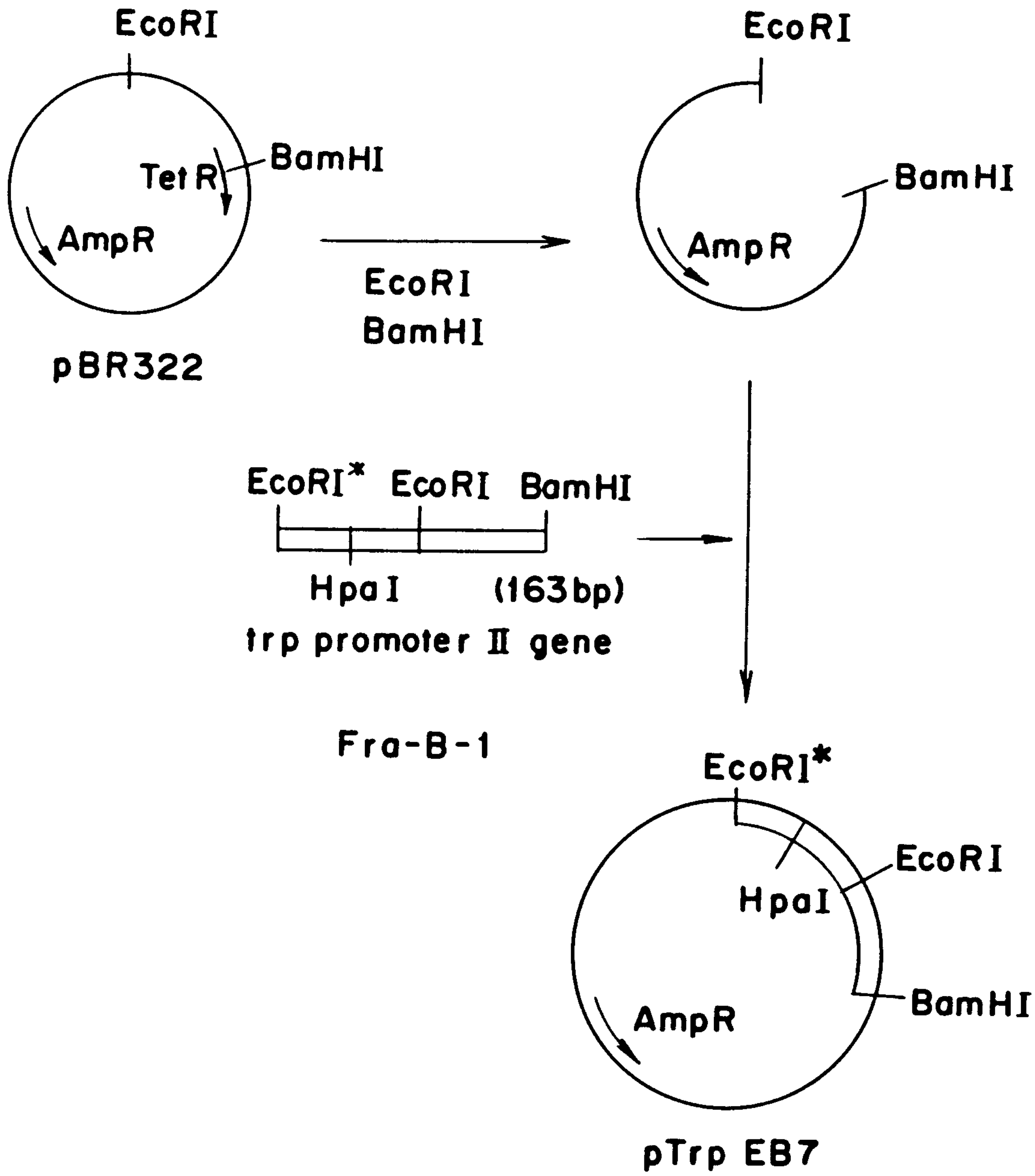
EcoRI
BamHI
Tet R
AmpR
pBR322
EcoRI
BamHI
EcoRI
BamHI
AmpR
EcoRI*
EcoRI
BamHI
HpaI
(163bp)
trp promoter II gene
Fra-B-1
EcoRI*
EcoRI
HpaI
BamHI
AmpR
pTrp EB7

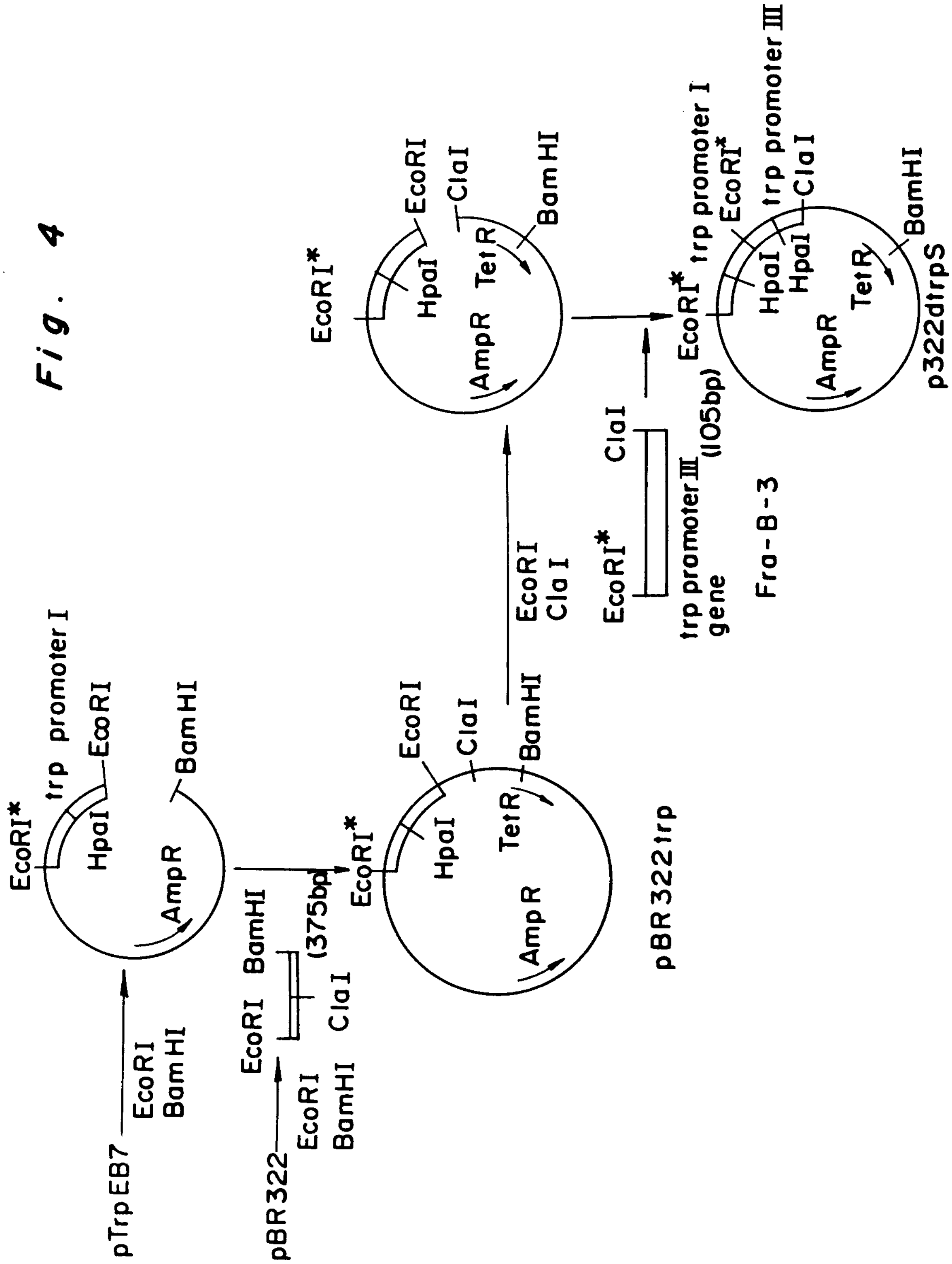
pTrpEB7
EcoRI
BamHI
EcoRI*
trp promoter I
EcoRI
HpaI
BamHI
AmpR
pBR322
EcoRI
BamHI
EcoRI BamHI
ClaI
(375bp)
EcoRI*
HpaI
EcoRI
ClaI
BamHI
TetR
AmpR
pBR322trp
EcoRI
ClaI
EcoRI*
HpaI
EcoRI
ClaI
BamHI
TetR
AmpR
EcoRI*
ClaI
trp promoter III
(105bp)
trp promoter III gene
Fra-B-3
EcoRI*
trp promoter I
EcoRI*
trp promoter III
ClaI
HpaI
HpaI
BamHI
TetR
AmpR
p322dtrpS

Fig. 4

Fig. 5(1)

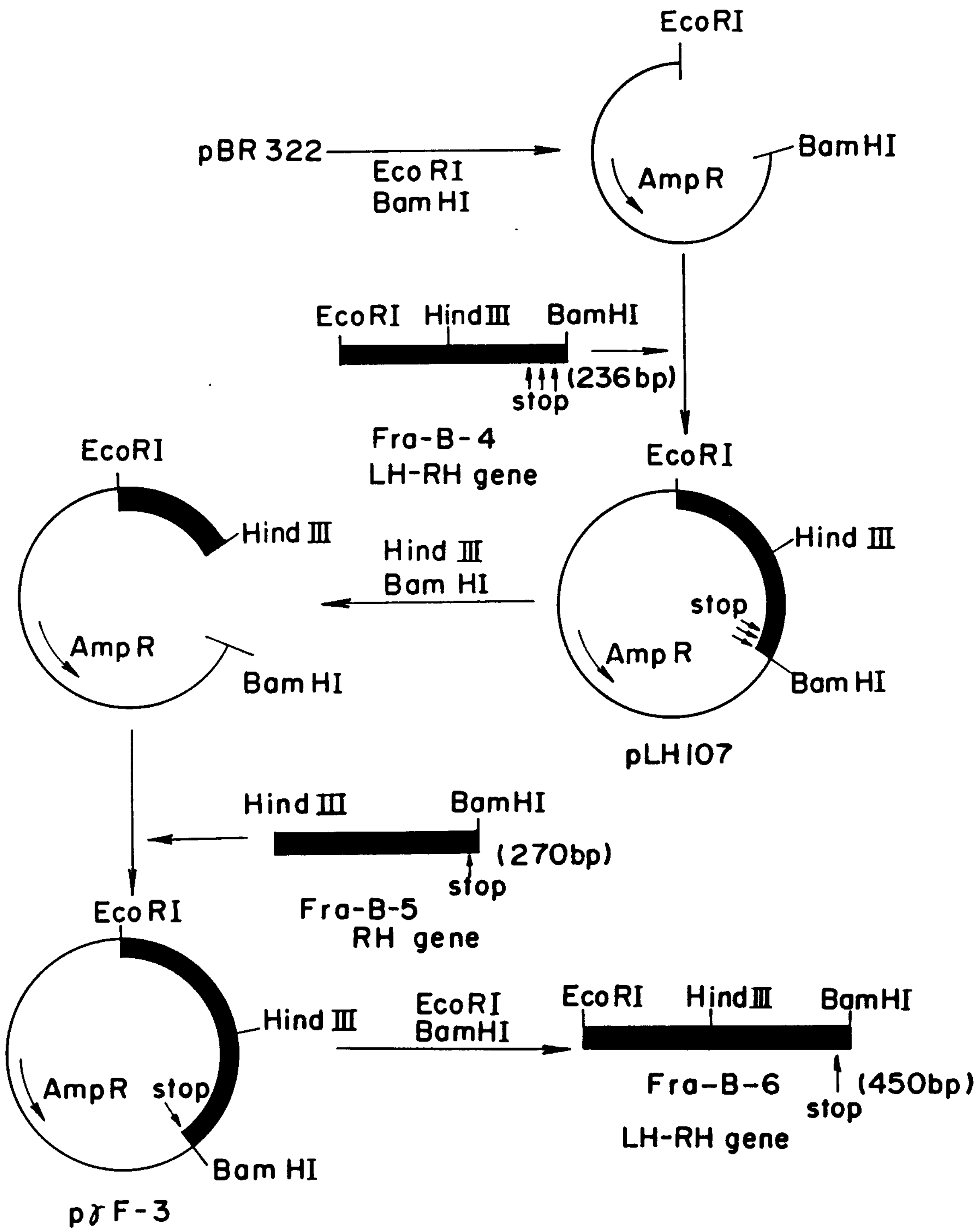
EcoRI
pBR322
Eco RI
Bam HI
AmpR
BamHI
EcoRI HindIII BamHI
(236bp)
stop
Fra-B-4
LH-RH gene
EcoRI
Hind III
Hind III
Bam HI
stop
AmpR
Bam HI
Amp R
Bam HI
pLH107
Hind III
BamHI
(270bp)
stop
Fra-B-5
RH gene
EcoRI
Hind III
EcoRI
BamHI
EcoRI HindIII BamHI
AmpR stop
Bam HI
Fra-B-6
(450bp)
stop
LH-RH gene
pγF-3

Fig. 5(2)

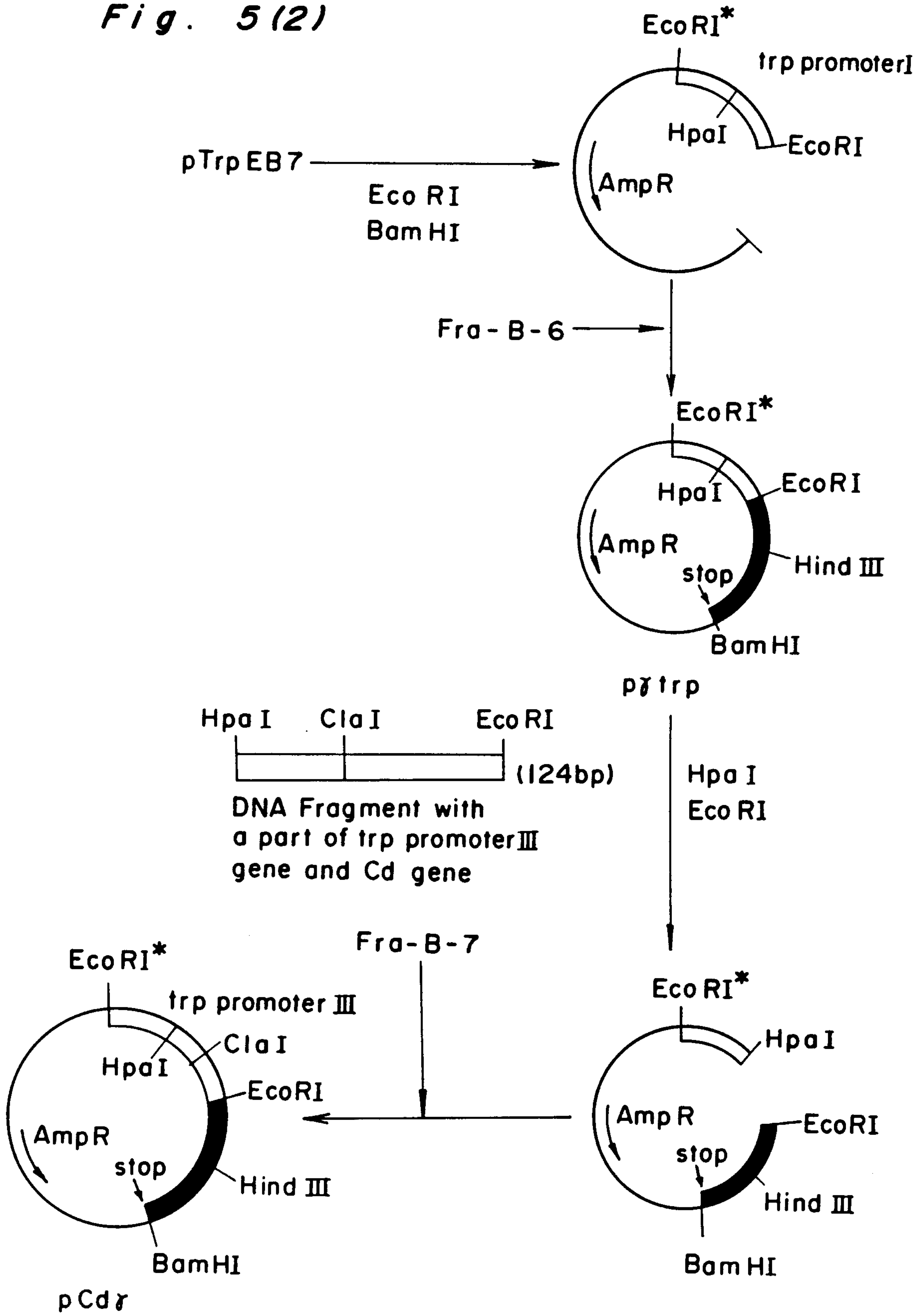
pTrpEB7
Eco RI
Bam HI
EcoRI*
trp promoterI
HpaI
EcoRI
AmpR
Fra-B-6
EcoRI*
HpaI
EcoRI
AmpR
stop
Hind III
BamHI
pγtrp
HpaI
EcoRI
HpaI
ClaI
EcoRI
(124bp)
DNA Fragment with
a part of trp promoterIII
gene and Cd gene
Fra-B-7
EcoRI*
HpaI
AmpR
EcoRI
stop
Hind III
BamHI
EcoRI*
trp promoter III
ClaI
HpaI
EcoRI
AmpR
stop
Hind III
BamHI
pCdγ

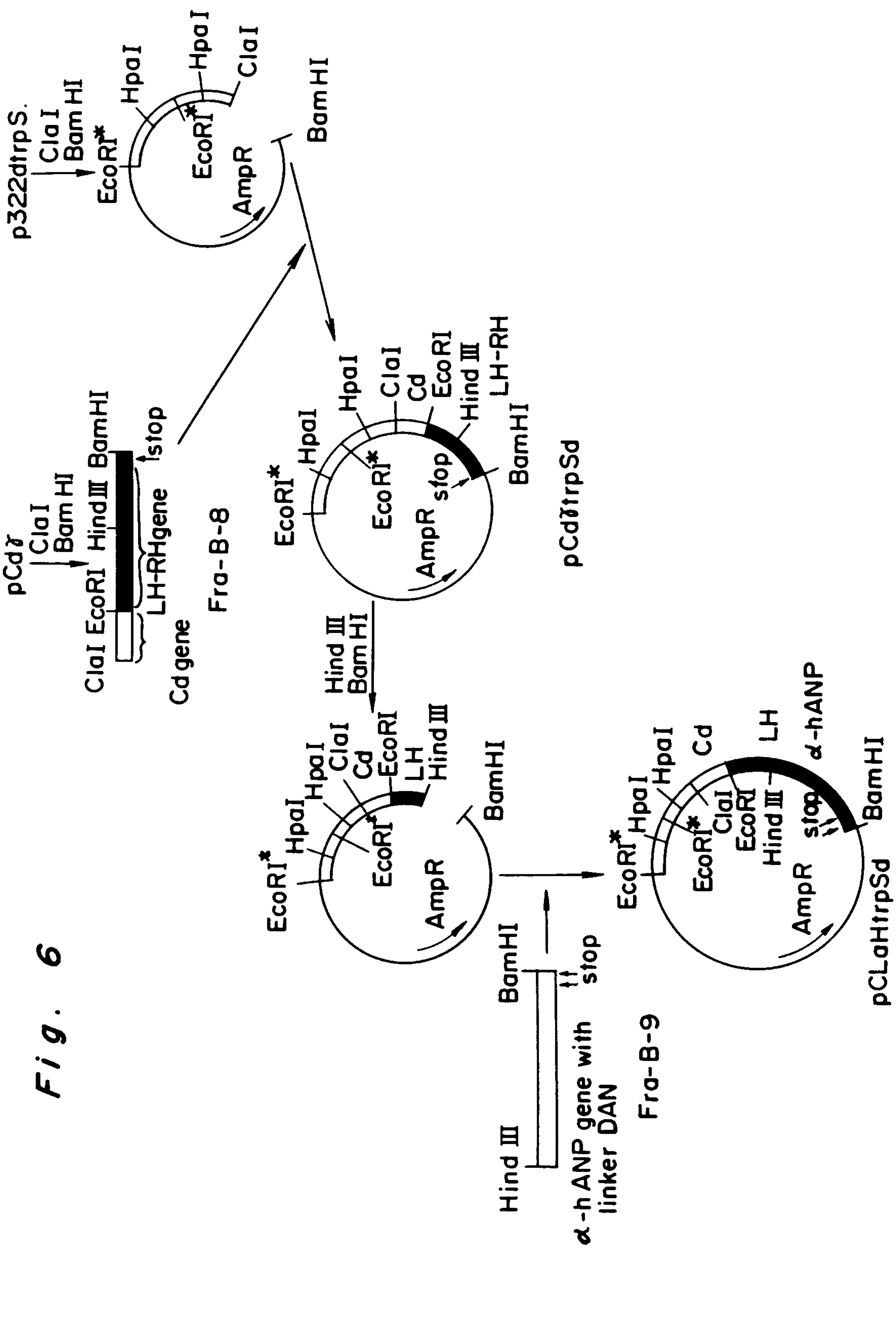
p322dtrpS
ClaI
BamHI
EcoRI*
HpaI
HpaI
ClaI
EcoRI*
AmpR
BamHI
pCdγ
ClaI
BamHI
ClaI EcoRI
HindIII
BamHI
LH-RHgene
stop
Cd gene
Fra-B-8
EcoRI*
HpaI
HpaI
ClaI
Cd
EcoRI
HindIII
LH-RH
EcoRI*
stop
AmpR
BamHI
pCdγtrpSd
HindIII
BamHI
EcoRI*
HpaI
HpaI
ClaI
Cd
EcoRI
LH
HindIII
EcoRI*
AmpR
BamHI
HindIII
BamHI
stop
α-hANP gene with
linker DAN
Fra-B-9
EcoRI*
HpaI
HpaI
Cd
LH
α-hANP
EcoRI*
ClaI
EcoRI
HindIII
stop
AmpR
BamHI
pCLaHtrpSd

Fig. 6

Fig. 7(1)

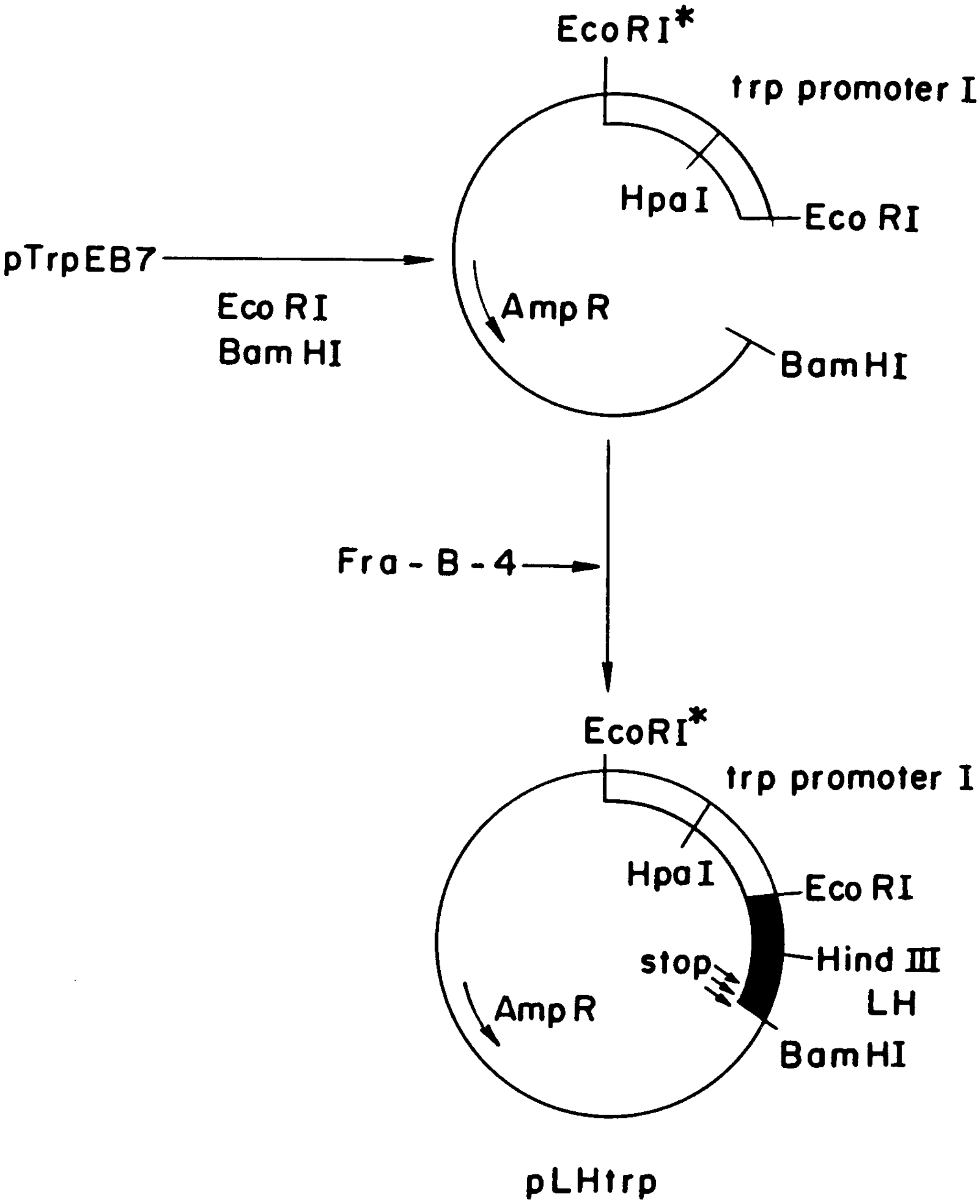
EcoRI*
trp promoter I
HpaI
Eco RI
pTrpEB7
Eco RI
Bam HI
Amp R
BamHI
Fra - B - 4
EcoRI*
trp promoter I
HpaI
Eco RI
stop
Hind III
LH
Amp R
BamHI
pLHtrp

*Fig. 7 (2)*

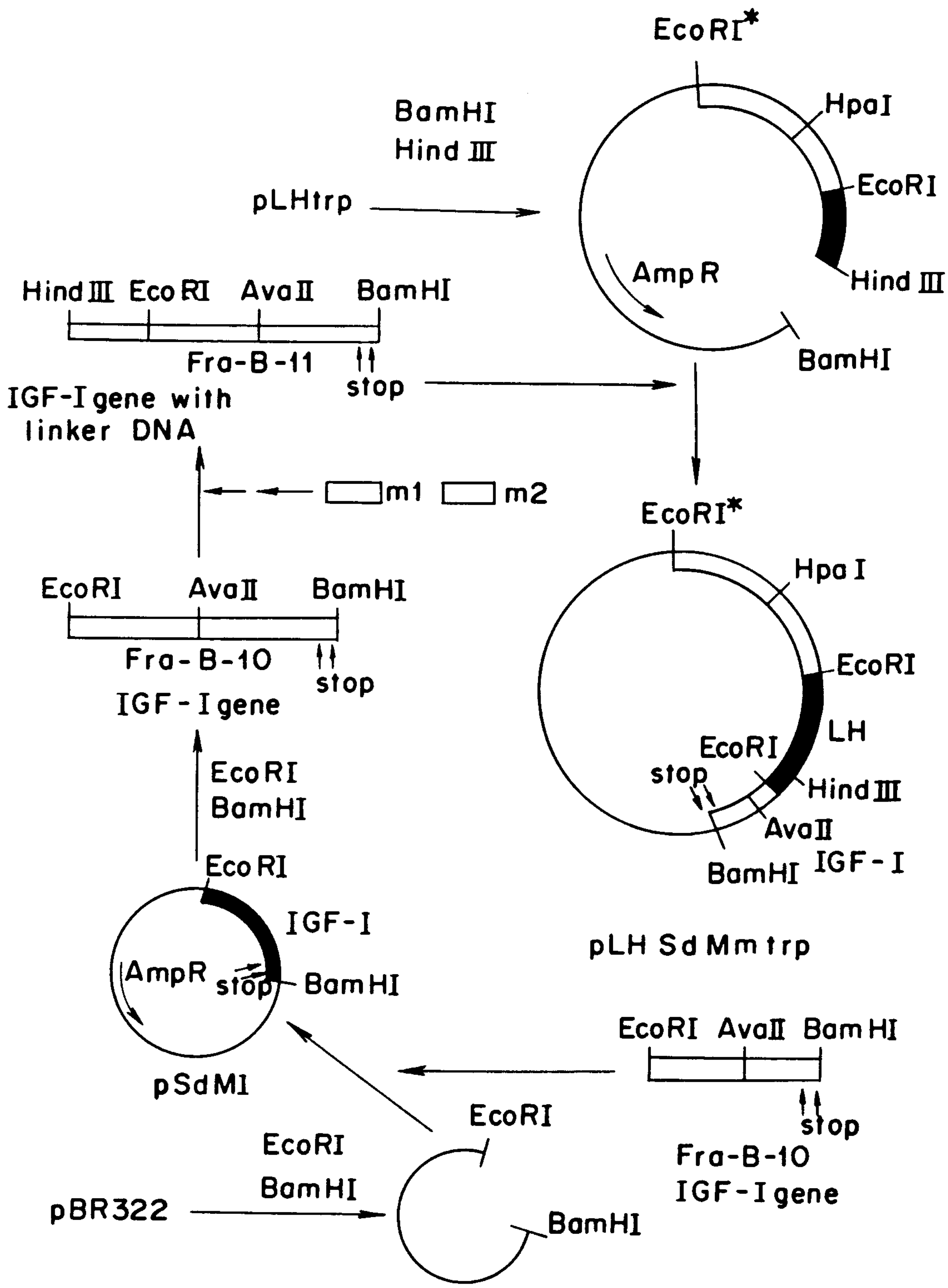
EcoRI*
BamHI
Hind III
pLHtrp
HpaI
EcoRI
Hind III
Amp R
BamHI
Hind III EcoRI AvaII BamHI
Fra-B-11
stop
IGF-I gene with linker DNA
m1
m2
EcoRI*
HpaI
EcoRI
LH
EcoRI
stop
Hind III
AvaII
BamHI
IGF-I
pLH SdMmtrp
EcoRI AvaII BamHI
Fra-B-10
stop
IGF-I gene
EcoRI
BamHI
EcoRI
IGF-I
AmpR
stop
BamHI
pSdMI
EcoRI AvaII BamHI
stop
Fra-B-10
IGF-I gene
EcoRI
EcoRI
BamHI
pBR322
BamHI

*Fig. 8*

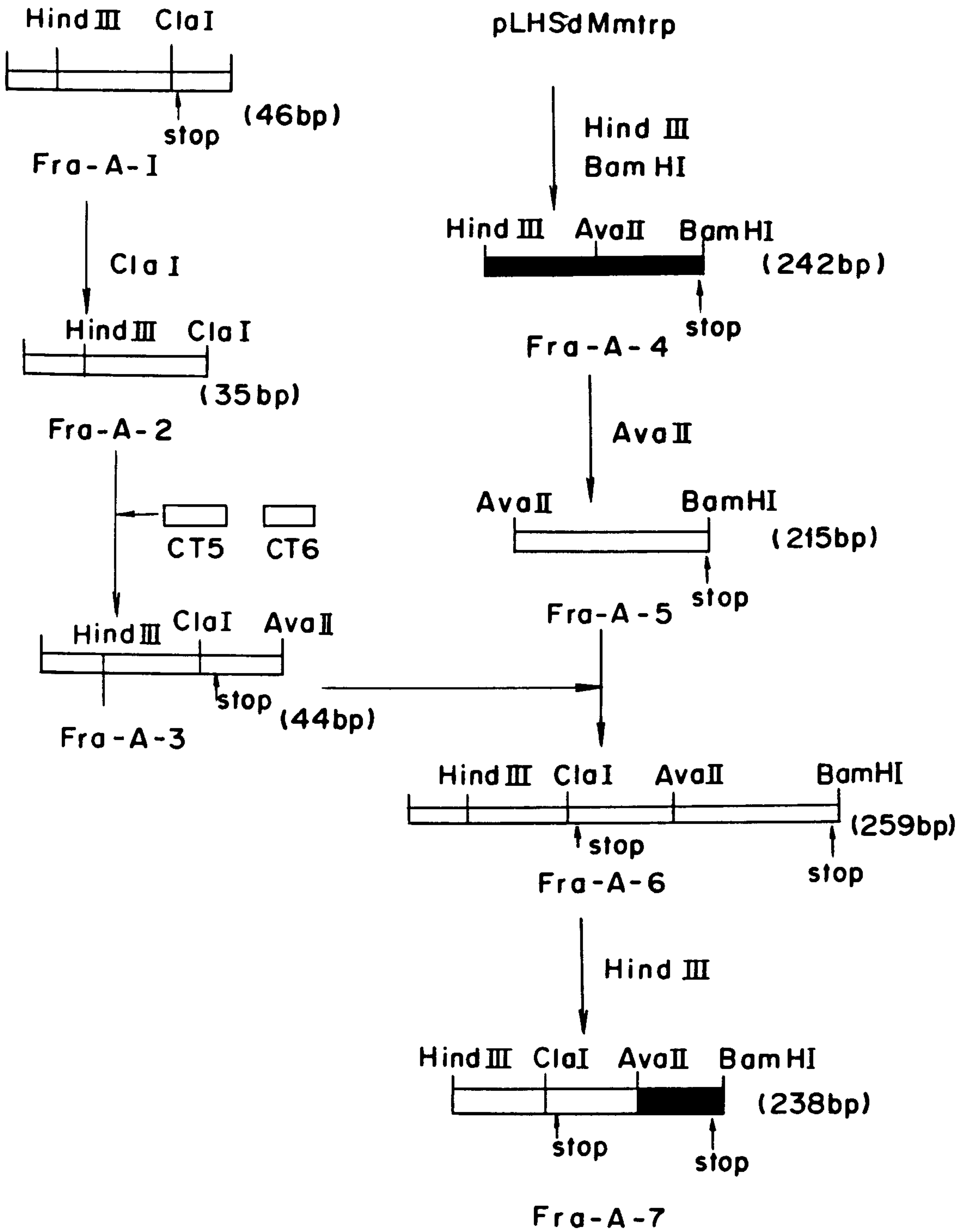
HindIII ClaI
(46bp)
stop
Fra-A-1
ClaI
HindIII ClaI
(35bp)
Fra-A-2
CT5 CT6
HindIII ClaI AvaII
stop (44bp)
Fra-A-3
pLHSdMmtrp
HindIII
BamHI
HindIII AvaII BamHI
(242bp)
stop
Fra-A-4
AvaII
AvaII BamHI
(215bp)
stop
Fra-A-5
HindIII ClaI AvaII BamHI
(259bp)
stop
stop
Fra-A-6
HindIII
HindIII ClaI AvaII BamHI
(238bp)
stop
stop
Fra-A-7

*Fig. 9*

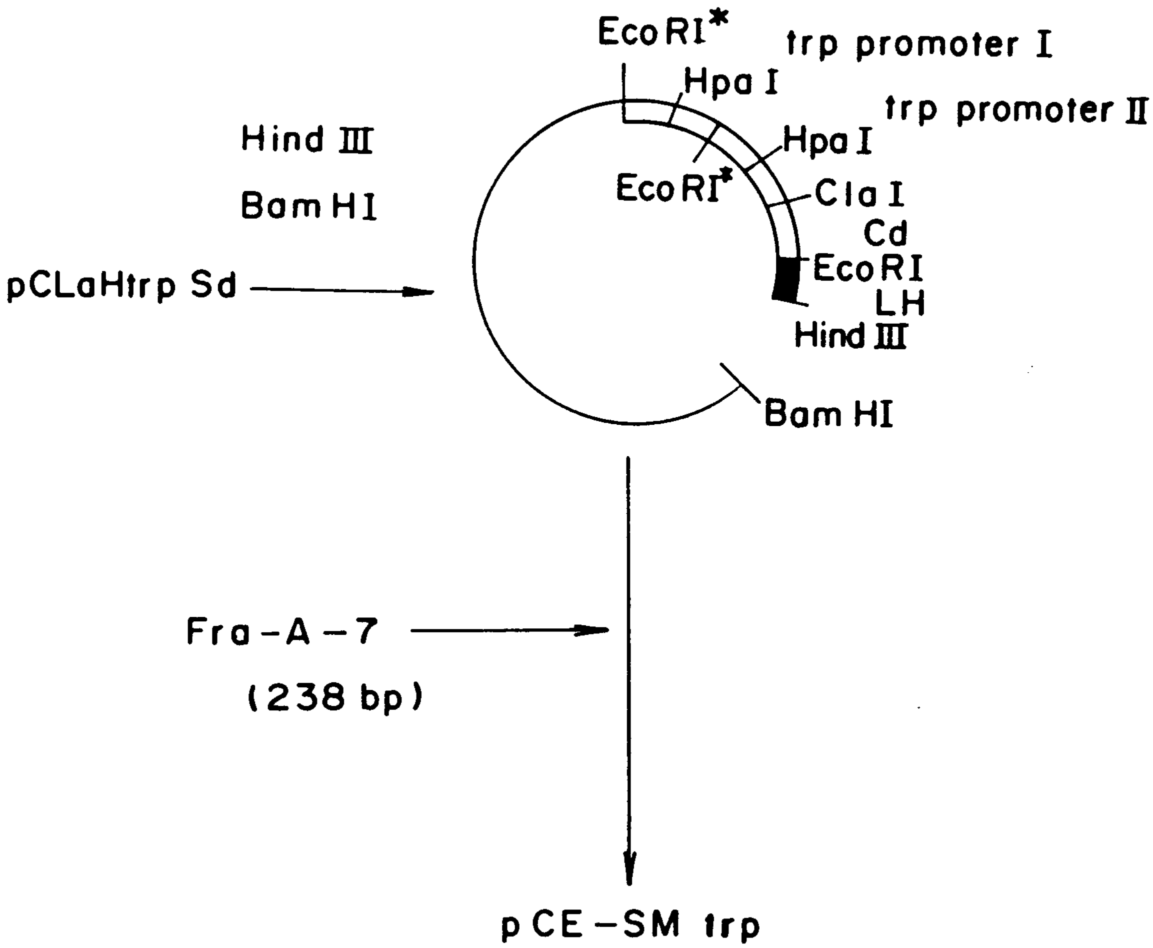
EcoRI*
trp promoter I
Hpa I
trp promoter II
Hind III
Hpa I
Bam HI
EcoRI*
Cla I
Cd
pCLaHtrp Sd
EcoRI
LH
Hind III
Bam HI
Fra-A-7
(238 bp)
pCE-SM trp

*Fig. 10*

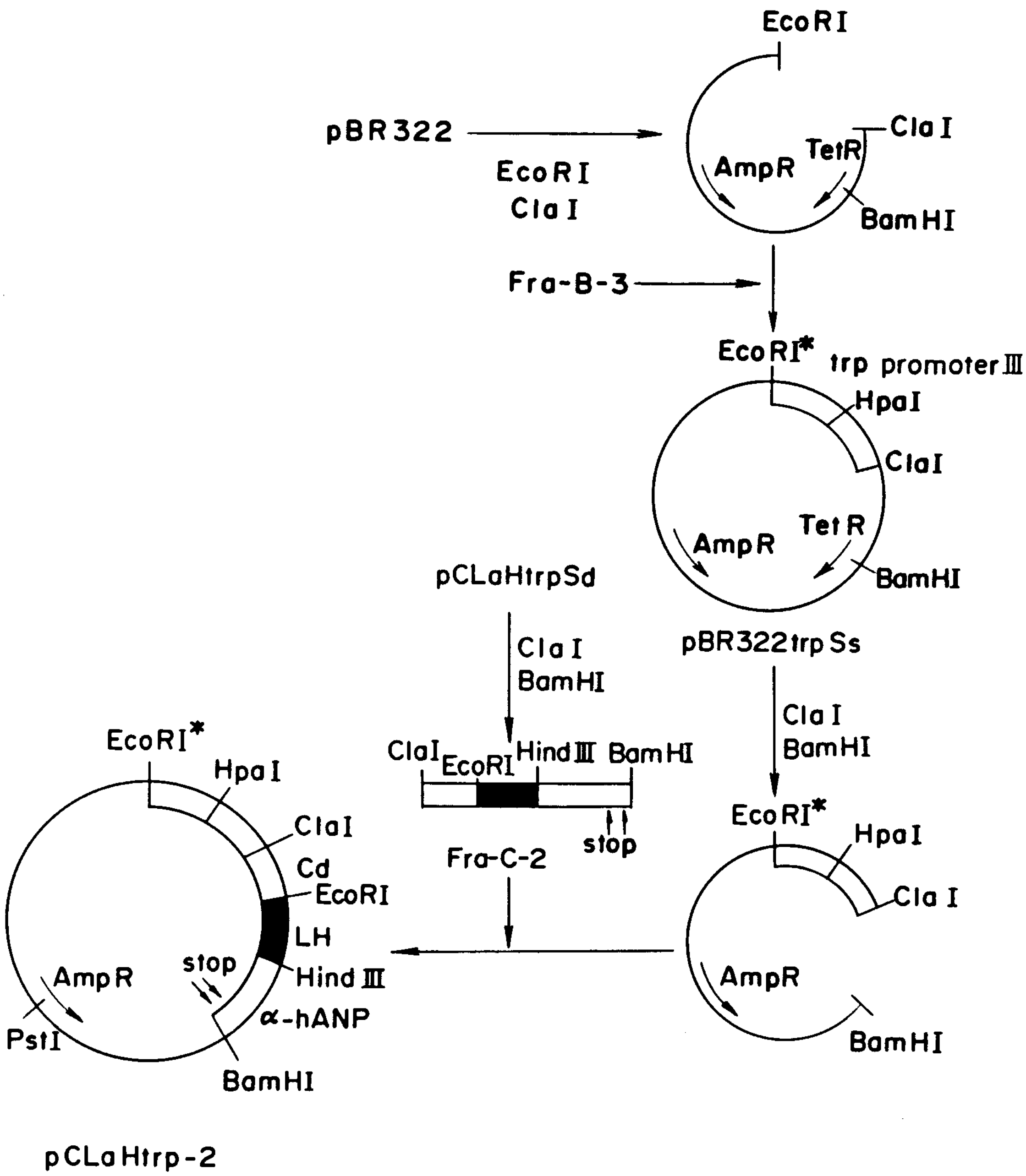
EcoRI
pBR322
EcoRI
ClaI
ClaI
TetR
AmpR
BamHI
Fra-B-3
EcoRI*
trp promoter III
HpaI
ClaI
TetR
AmpR
BamHI
pCLaHtrpSd
pBR322trpSs
ClaI
BamHI
ClaI
BamHI
EcoRI*
HpaI
ClaI
EcoRI
HindIII
BamHI
stop
Fra-C-2
EcoRI*
HpaI
ClaI
AmpR
BamHI
ClaI
Cd
EcoRI
LH
HindIII
stop
AmpR
PstI
α-hANP
BamHI
pCLaHtrp-2

Fig. 11

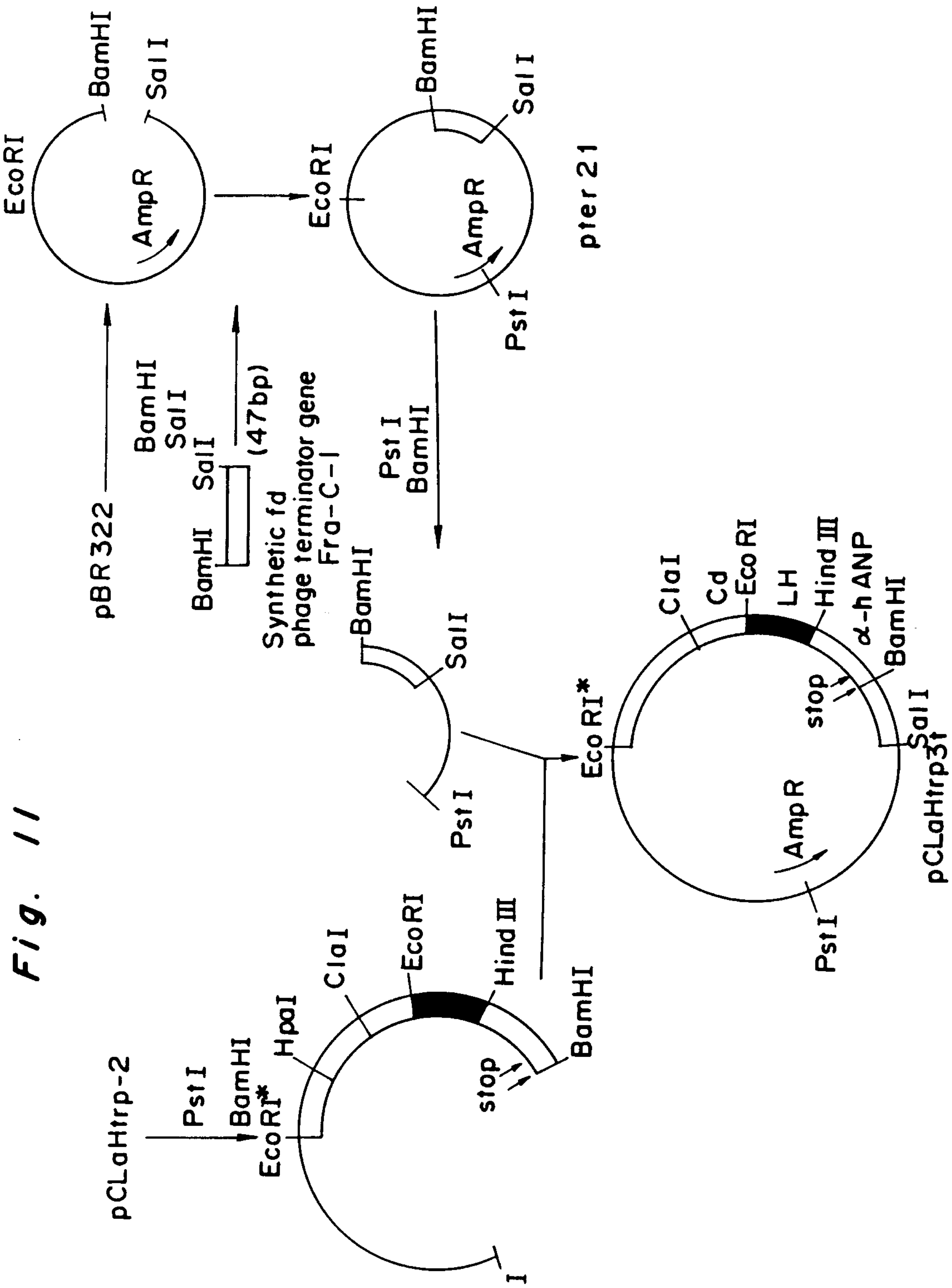
pBR322
EcoRI
BamHI
SalI
AmpR
BamHI
SalI
BamHI SalI
(47bp)
Synthetic fd
phage terminator gene
Fra-C-I
EcoRI
BamHI
SalI
AmpR
PstI
pter21
PstI
BamHI
BamHI
SalI
PstI
pCLaHtrp-2
PstI
BamHI
EcoRI*
HpaI
ClaI
EcoRI
Hind III
stop
BamHI
PstI
EcoRI*
ClaI
Cd
EcoRI
LH
Hind III
α-h ANP
BamHI
stop
SalI
AmpR
PstI
pCLaHtrp3t

Fig. 12

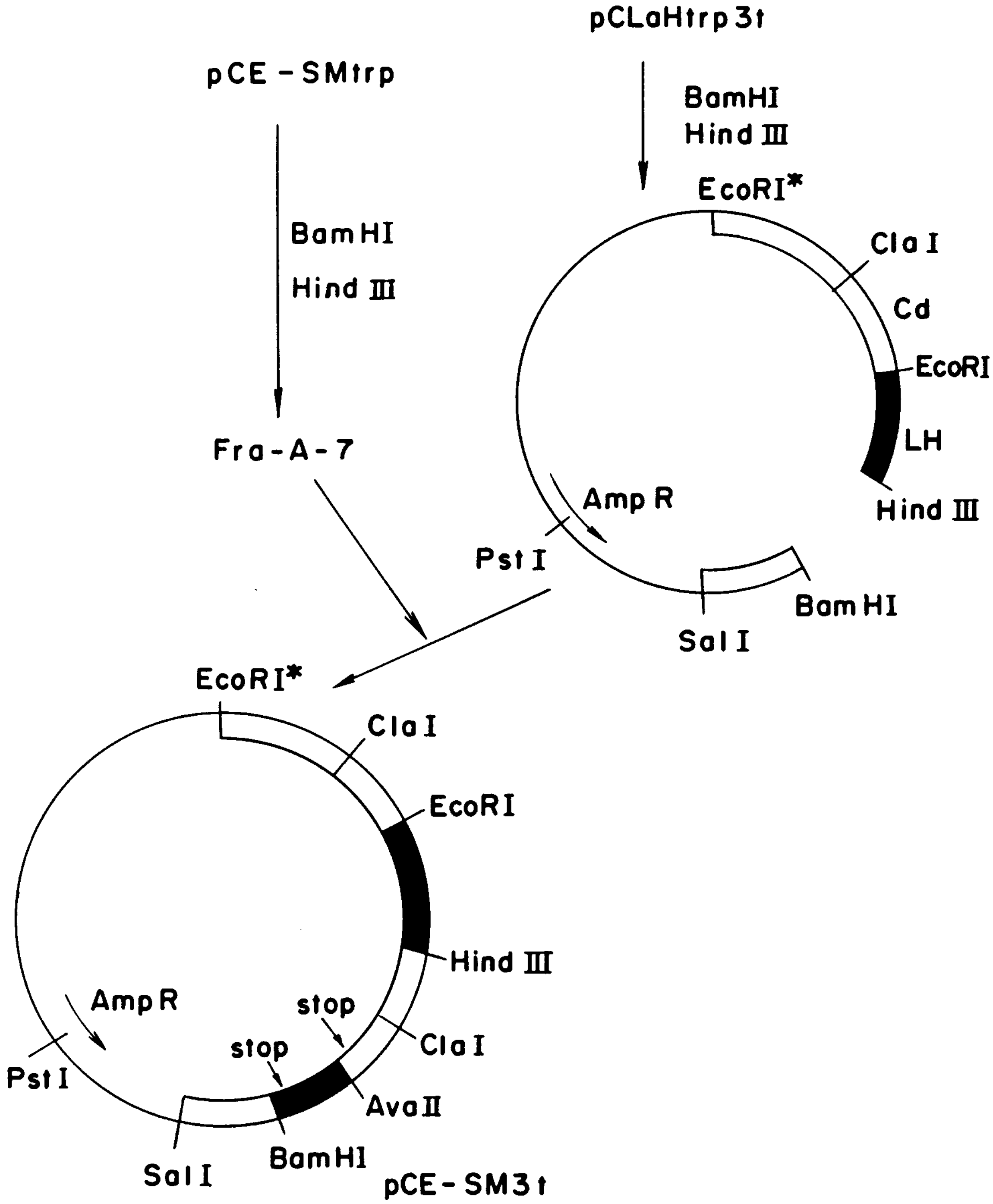
pCLaHtrp3t
BamHI
Hind III
EcoRI*
ClaI
Cd
EcoRI
LH
Hind III
AmpR
PstI
BamHI
SalI
pCE-SMtrp
BamHI
Hind III
Fra-A-7
EcoRI*
ClaI
EcoRI
Hind III
AmpR
stop
stop
ClaI
PstI
AvaII
SalI
BamHI
pCE-SM3t

FFig. 13 (1)

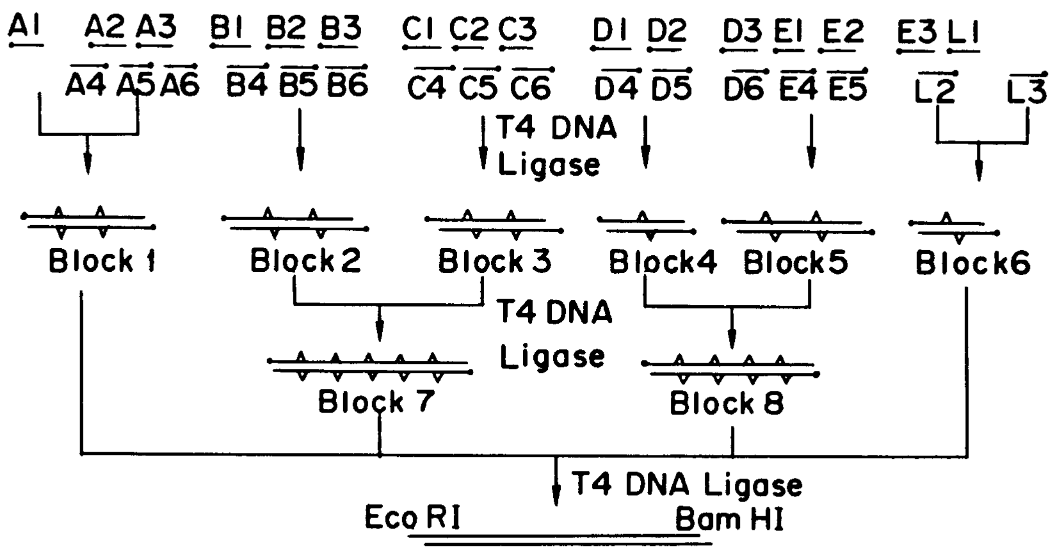


Fra-B-4

Fig. 13(2)

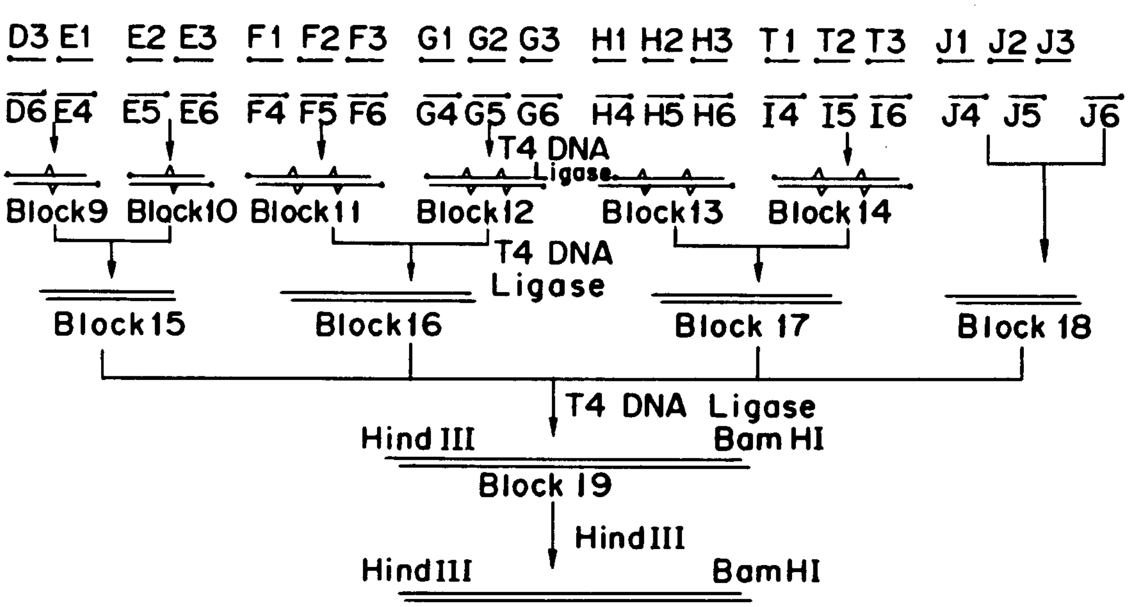


Fra-B-5

Fig. 14

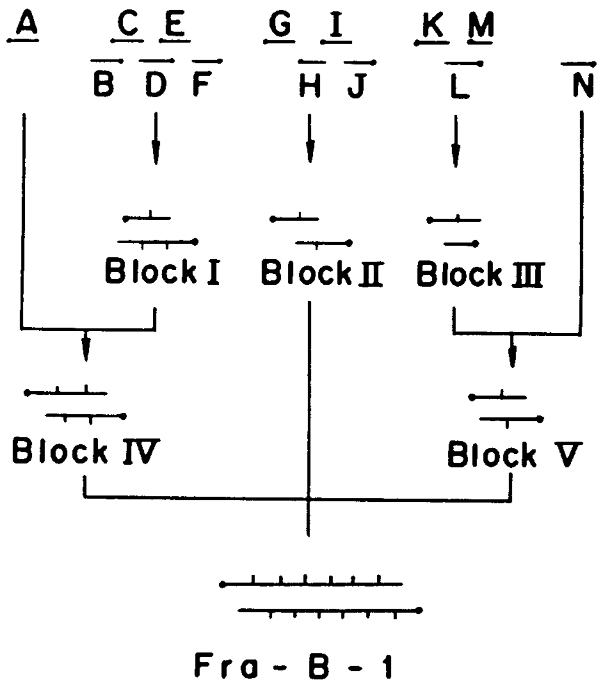
A
C E
G I
K M
B D F
H J
L
N
Block I
Block II
Block III
Block IV
Block V
Fra - B - 1

Fig. 15

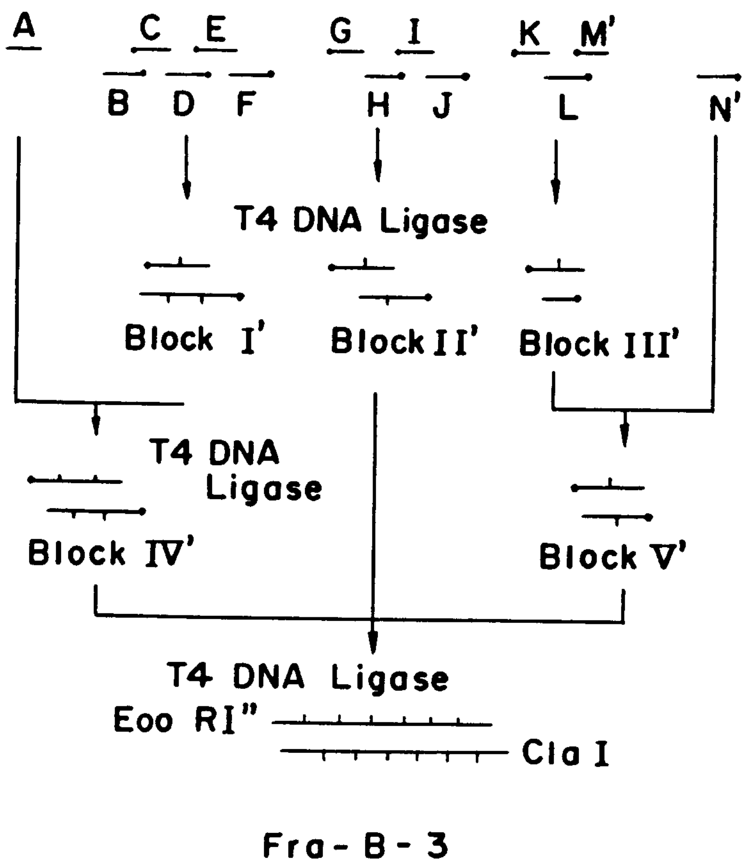
A
C E
G I
K M'
B D F
H J
L
N'
T4 DNA Ligase
Block I'
Block II'
Block III'
T4 DNA Ligase
Block IV'
Block V'
T4 DNA Ligase
Eoo RI"
Cla I
Fra - B - 3

Fig. 16

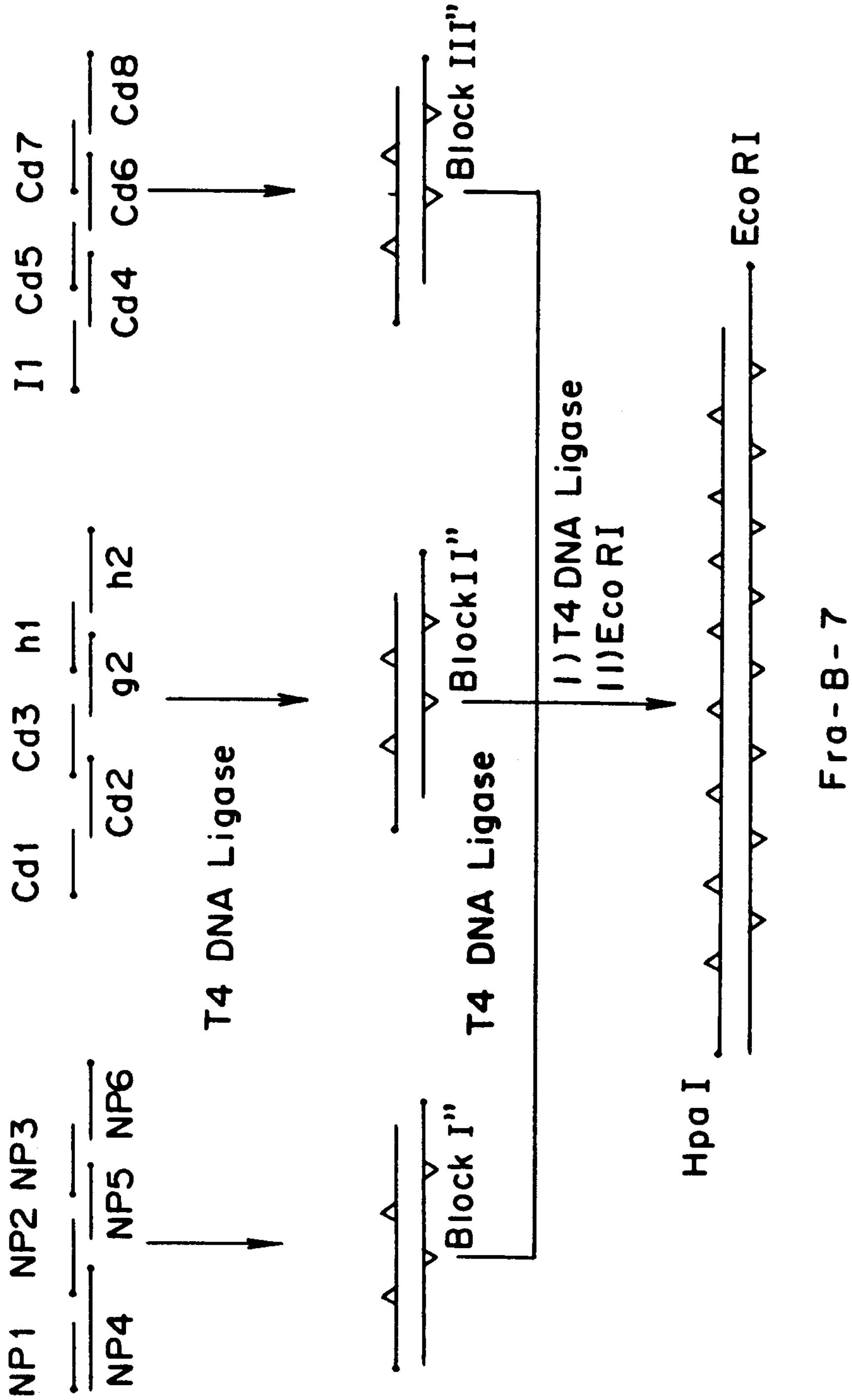
NP1 NP2 NP3
NP4 NP5 NP6
Block I"
Cd1 Cd3 h1
Cd2 g2 h2
T4 DNA Ligase
Block II"
I1 Cd5 Cd7
Cd4 Cd6 Cd8
Block III"
T4 DNA Ligase
I) T4 DNA Ligase
II) Eco RI
Hpa I
Eco RI
Fra-B-7

Fig. 17

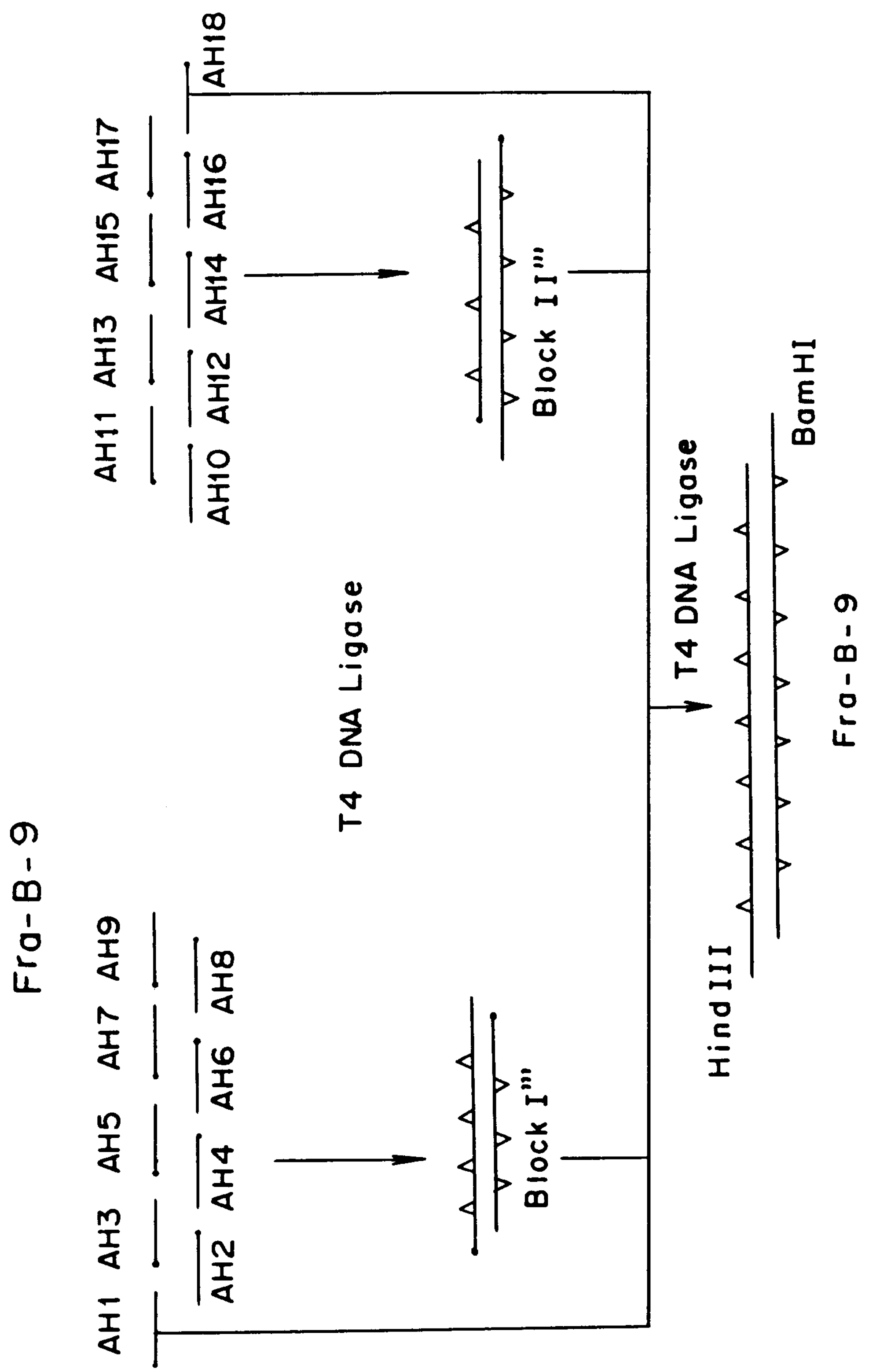
Fra-B-9
AH1 AH3 AH5 AH7 AH9
AH2 AH4 AH6 AH8
Block I'''
AH11 AH13 AH15 AH17
AH10 AH12 AH14 AH16 AH18
Block II'''
T4 DNA Ligase
T4 DNA Ligase
HindIII
BamHI
Fra-B-9

Fig. 18

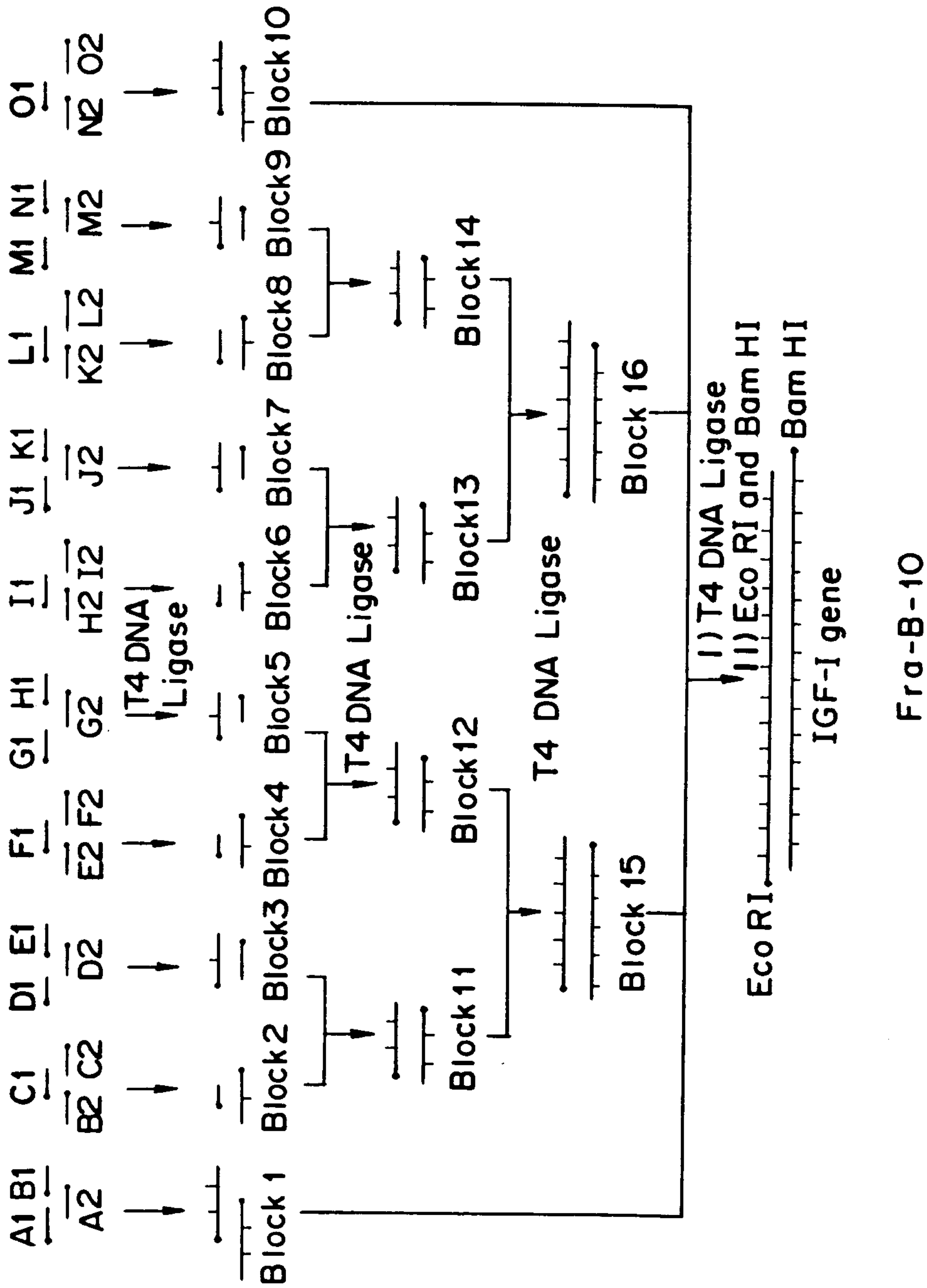
A1 B1
A2
C1
B2 C2
D1 E1
D2
F1
E2 F2
G1 H1
G2
I1
H2 I2
J1 K1
J2
L1
K2 L2
M1 N1
M2
O1
N2 O2
T4 DNA Ligase
Block 1
Block 2
Block 3
Block 4
Block 5
Block 6
Block 7
Block 8
Block 9
Block 10
T4 DNA Ligase
T4 DNA Ligase
Block 11
Block 12
Block 13
Block 14
T4 DNA Ligase
Block 15
Block 16
I) T4 DNA Ligase
II) Eco RI and Bam HI
Eco RI
Bam HI
IGF-I gene
Fra-B-10